(19) Europäisches Patentamt
European Patent Office
Office européen des brevets

(11) EP 2 714 052 B1

(12) **EUROPEAN PATENT SPECIFICATION**

(45) Date of publication and mention of the grant of the patent:
**19.09.2018 Bulletin 2018/38**

(21) Application number: **12724631.2**

(22) Date of filing: **31.05.2012**

(51) Int Cl.:
*A61K 31/7034* (2006.01) *A61K 31/551* (2006.01)
*A61K 31/4515* (2006.01) *A61P 3/04* (2006.01)
*A61P 3/06* (2006.01) *A61P 3/08* (2006.01)

(86) International application number:
**PCT/EP2012/060194**

(87) International publication number:
**WO 2012/163990 (06.12.2012 Gazette 2012/49)**

(54) **SGLT-2 INHIBITORS FOR TREATING METABOLIC DISORDERS IN PATIENTS TREATED WITH NEUROLEPTIC AGENTS**

SGLT-2 INHIBITOREN ZUR BEHANDLUNG VON STOFFWECHSELERKRANKUNGEN BEI PATIENTEN MIT NEUROLEPTIKA

INHIBITEURS DE SGLT-2 POUR TRAITER DES TROUBLES MÉTABOLIQUES CHEZ DES PATIENTS TRAITÉS AVEC DES AGENTS NEUROLEPTIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.06.2011 EP 11168641**
**30.01.2012 EP 12153052**

(43) Date of publication of application:
**09.04.2014 Bulletin 2014/15**

(73) Proprietor: **Boehringer Ingelheim International GmbH**
**55216 Ingelheim am Rhein (DE)**

(72) Inventors:
- **WIENRICH, Marion**
**55216 Ingelheim Am Rhein (DE)**
- **MAYOUX, Eric**
**55216 Ingelheim Am Rhein (DE)**

(74) Representative: **Simon, Elke Anna Maria et al**
**Boehringer Ingelheim GmbH**
**Binger Strasse 173**
**55216 Ingelheim am Rhein (DE)**

(56) References cited:
**WO-A1-2008/089892 WO-A1-2010/092123**
**WO-A2-2008/055940 WO-A2-2008/116195**

- **MAAYAN LAWRENCE ET AL: "Effectiveness of medications used to attenuate antipsychotic-related weight gain and metabolic abnormalities: a systematic review and meta-analysis.", NEUROPSYCHOPHARMACOLOGY : OFFICIAL PUBLICATION OF THE AMERICAN COLLEGE OF NEUROPSYCHOPHARMACOLOGY JUN 2010 LNKD- PUBMED:20336059, vol. 35, no. 7, June 2010 (2010-06), pages 1520-1530, XP002679348, ISSN: 1740-634X**
- **ELLINGER LARA K ET AL: "Efficacy of Metformin and Topiramate in Prevention and Treatment of Second-Generation Antipsychotic-Induced Weight Gain", ANNALS OF PHARMACOTHERAPY, HARVEY WHITNEY BOOKS COMPANY, vol. 44, no. 4, 1 April 2010 (2010-04-01), pages 668-679, XP009156007, ISSN: 1060-0280**
- **GOODWIN NICOLE C ET AL: "Novel L-xylose derivatives as selective sodium-dependent glucose cotransporter 2 (SGLT2) inhibitors for the treatment of type 2 diabetes.", JOURNAL OF MEDICINAL CHEMISTRY 22 OCT 2009 LNKD-PUBMED:19785435, vol. 52, no. 20, 22 October 2009 (2009-10-22), pages 6201-6204, XP002679349, ISSN: 1520-4804**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Technical Field of the Invention

The invention relates to a SGLT2 inhibitor for use in a method for improving glycemic control in a patient treated for a psychotic disorder with a neuroleptic agent, characterized in that the SGLT2 inhibitor is selected from the group consisting of dapagliflozin, canagliflozin and empagliflozin and in that the neuroleptic agent and the SGLT2 inhibitor are administered to the patient. Furthermore the invention relates to a pharmaceutical composition comprising (a) a neuroleptic agent and (b) an SGLT2 inhibitor selected from the group consisting of dapagliflozin, canagliflozin and empagliflozin.

### Background of the Invention

Neuroleptics (also called antipsychotics) are drugs that modify psychotic symptoms, including symptoms of schizophrenia, delusional disorder and psychotic depression. Some types of neuroleptic drugs are also used to treat non-psychosis disorders such as Tourette syndrome and Asperger syndrome. There are two classes of neuroleptic drugs: typical antipsychotics, which were discovered and first used in the 1950s, and atypical antipsychotics, which were developed in and used since the 1970s. Atypical neuroleptic drugs generally are regarded as more effective and less likely to cause side effects such as Extrapyramidal Syndrome (EPS) than typical neuroleptic drugs. Studies indicate that psychotic episodes are linked to an excess of a neurotransmitter called dopamine. Both typical and atypical neuroleptic drugs work by blocking dopamine receptors in the brain, reducing the activity of dopamine and thus reducing psychosis. Although both classes of drugs work in similar ways, it has been noted that typical antipsychotic drugs are less selective in the types of dopamine receptors they block. It has been suggested that this lack of selectivity is responsible for the increased range and severity of side effects caused by typical neuroleptic drugs, in particular EPS.
Neuroleptic agents comprise a group of the following 7 classes of drugs: Phenothiazines, further divided into the aliphatics, piperidines, and piperazines, Thioxanthenes (eg, droperidol), Butyrophenones (eg, haloperidol), Dibenzoxazepines (eg, loxapine), Dihydroindolone (eg, molindone), Diphenylbutylpiperidine (eg, pimozide), Benzisoxazole (eg, risperidone).
Metabolic side effects are among the undesired side effects observed with the use of neuroleptic agents, in particular atypical neuroleptic agents. These side effects include glucose dysregulation, insuline resistance, hyperlipidemia, weight gain and hypertension and can put the patients at risk of cardiometabolic disorders (see for example Boyda et al. (2000) Trends in Pharmacological Sciences 31: 484-497).
There is therefore a need for methods, medicaments and pharmaceutical compositions which allow to treat the psychotic disorders effectively, while reducing or avoiding the side effects associated with the antipsychotic treaments, in particular, metabolic side effects.

### Aim of the present invention

Another aim of the present invention is to provide a SGLT2 inhibitor for use in methods and pharmaceutical compositions for improving glycemic control in a patient treated for a psychotic disorder, in particular in a patient treated with a neuroleptic agent.
Further aims of the present invention become apparent to the one skilled in the art by description hereinbefore and in the following and by the examples.

### Summary of the Invention

The present invention addresses the above aims and needs by providing a SGLT2 inhibitor for use in a a method for improving glycemic control in a patient treated for a psychotic disorder with a neuroleptic agent, characterized in that the SGLT2 inhibitor is selected from the group consisting of dapagliflozin, canagliflozin and empagliflozin and in that the neuroleptic agent and the SGLT2 inhibitor are administered to the patient. The present invention also addresses the above aims and needs by providing pharmaceutical compositions comprising a neuroleptic agent and an SGLT-2 inhibitor selected from the group consisting of dapagliflozin, canagliflozin and empagliflozin.
SGLT2 inhibitors represent a novel class of agents that are being developed for the treatment or improvement of glycemic control in patients with type 2 diabetes. Examples of SGLT-2 inhibitors are glucopyranosyl-substituted benzene derivatives, for example as described in WO 01/27128, WO 03/099836, WO 2005/092877, WO 2006/034489, WO 2006/064033, WO 2006/117359, WO 2006/117360, WO 2007/025943, WO 2007/028814, WO 2007/031548, WO 2007/093610, WO 2007/128749, WO 2008/049923, WO 2008/055870, WO 2008/055940. The glucopyranosyl-substituted benzene derivatives are proposed as inducers of urinary sugar excretion and as medicaments in the treatment of diabetes.

Accordingly, in one embodiment, the present invention provides a SGLT2 inhibitor for use in a method for improving glycemic control in a patient treated for a psychotic disorder with a neuroleptic agent, characterized in that the SGLT2 inhibitor is selected from the group consisting of dapagliflozin, canagliflozin and empagliflozin and in that the neuroleptic agent and the SGLT2 inhibitor are administered to the patient.

In one aspect, the SGLT2 inhibitor is 1-chloro-4-(β-D-glucopyranos-1-yl)-2-[4-((*S*)-tetrahydrofuran-3-yloxy)-benzyl]-benzene, also called empagliflozin.

In one aspect, the SGLT-2 inhibitor is dapagliflozin or canagliflozin..

In one aspect, the neuroleptic agent is a typical neuroleptic agent or an atypical neuroleptic agent.

In one aspect, the neuroleptic agent is a Phenothiazine, a Thioxanthene, a Butyrophenone, a Dibenzoxazepine, a Dihydroindolone, a Diphenylbutylpiperidine, or a Benzisoxazole.

In one aspect, the neuroleptic agent is olanzapine, risperidone, quetiapine (or quetiapine fumarate), amisulpiride, aripiprazole, haloperidol, clozapine, ziprasidone, zotepine, paliperidone or osanetant. In one aspect, the neuroleptic agent is olanzapine. In one aspect, the neuroleptic agent is clozapine.

In one aspect, the SGLT-2 inhibitor and the neuroleptic agent are administered in combination or alternation or sequentially to the patient.

In one aspect, the patient:

(1) is an individual diagnosed of one or more of the conditions selected from the group consisting of overweight, obesity, visceral obesity and abdominal obesity; or

(2) is an individual who shows one, two or more of the following conditions:

(a) a fasting blood glucose or serum glucose concentration greater than 100 mg/dL, in particular greater than 125 mg/dL;

(b) a postprandial plasma glucose equal to or greater than 140 mg/dL;

(c) an HbA1c value equal to or greater than 6.5 %, in particular equal to or greater than 8.0 %; or

(3) is an individual wherein one, two, three or more of the following conditions are present:

(a) obesity, visceral obesity and/or abdominal obesity,

(b) triglyceride blood level ≥ 150 mg/dL,

(c) HDL-cholesterol blood level < 40 mg/dL in female patients and < 50 mg/dL in male patients,

(d) a systolic blood pressure ≥ 130 mm Hg and a diastolic blood pressure ≥ 85 mm Hg,

(e) a fasting blood glucose level ≥ 100 mg/dL.

In a further embodiment, the present invention provides a pharmaceutical composition comprising (a) an SGLT2 inhibitor, and (b) a neuroleptic agent.

In one aspect, the SGLT2 inhibitor is 1-chloro-4-(β-D-glucopyranos-1-yl)-2-[4-((*S*)-tetrahydrofuran-3-yloxy)-benzyl]-benzene, also called empagliflozin.

In one aspect, the SGLT-2 inhibitor is dapagliflozin or canagliflozin.

In one aspect, the neuroleptic agent is a typical neuroleptic agent or an atypical neuroleptic agent.

In one aspect, the neuroleptic agent is a Phenothiazine, a Thioxanthene, a Butyrophenone, a Dibenzoxazepine, a Dihydroindolone, a Diphenylbutylpiperidine, or a Benzisoxazole.

In one aspect, the neuroleptic agent is olanzapine, risperidone, quetiapine (quetiapine fumarate), amisulpiride, aripiprazole, haloperidol, clozapine, ziprasidone, zotepine, paliperidone or osanetant. In one aspect, the neuroleptic agent is olanzapine. In one aspect, the neuroleptic agent is clozapine.

In one aspect, the composition is suitable for combined or simultaneous or sequential use of the SGLT2 inhibitor and the neuroleptic agent.

In one aspect, a psychotic disorder hereinabove and hereinafter is schizophrenia. In one aspect, a patient hereinabove and hereinafter is a subject treated for a psychotic disorder, for example schizophrenia.

In another aspect, a patient in the context of the present invention is a subject is treated for manic episodes associated with bipolar I disorder. In another aspect, a patient is a subject is treated for mixed episodes associated with bipolar I disorder. In one other aspect, a patient is a subject is treated for manic or mixed episodes associated with bipolar I disorder. In another aspect, a patient is a subject is treated for acute agitation associated with schizophrenia and bipolar I mania. In another aspect, a patient is a subject is treated for depressive episodes associated with bipolar I disorder. In another apsect, a patient is a subject is treated for depression.

## Definitions

The term **"neuroleptic agent"** or **"antipsychotic agent"** according to the present invention means a tranquilizing but not sedating psychiatric medication primarily used to manage psychosis including delusions, hallucinations or disordered thought, particular in conditions such as schizophrenia.

The term **"psychotic disorder"** or **"psychosis"** according to the present invention means an abnormal condition of the mind. It is a generic psychiatric term for a mental state often described as involving a "loss of contact with reality". The term psychosis is given to the more severe forms of psychiatric disorder, during which hallucinations and delusions and impaired insight may occur. Subjects experiencing psychosis may report hallucinations or delusional beliefs, and may exhibit personality changes and thought disorder. Depending on its severity, this may be accompanied by unusual or bizarre behavior, as well as difficulty with social interaction and impairment in carrying out the daily life activities.

The term **"active ingredient"** of a pharmaceutical composition according to the present invention means the SGLT2 inhibitor and/or neuroleptic agent according to the present invention.

The term **"body mass index"** or **"BMI"** of a human patient is defined as the weight in kilograms divided by the square of the height in meters, such that BMI has units of $kg/m^2$.

The term **"overweight"** is defined as the condition wherein the individual has a BMI greater than or 25 $kg/m^2$ and less than 30 $kg/m^2$. The terms "overweight" and "pre-obese" are used interchangeably.

The term **"obesity"** is defined as the condition wherein the individual has a BMI equal to or greater than 30 $kg/m^2$. According to a WHO definition the term obesity may be categorized as follows: the term "class I obesity" is the condition wherein the BMI is equal to or greater than 30 $kg/m^2$ but lower than 35 $kg/m^2$; the term "class II obesity" is the condition wherein the BMI is equal to or greater than 35 $kg/m^2$ but lower than 40 $kg/m^2$; the term "class III obesity" is the condition wherein the BMI is equal to or greater than 40 $kg/m^2$.

The term **"visceral obesity"** is defined as the condition wherein a waist-to-hip ratio of greater than or equal to 1.0 in men and 0.8 in women is measured. It defines the risk for insulin resistance and the development of pre-diabetes.

The term **"abdominal obesity"** is usually defined as the condition wherein the waist circumference is > 40 inches or 102 cm in men, and is > 35 inches or 94 cm in women. With regard to a Japanese ethnicity or Japanese patients abdominal obesity may be defined as waist circumference ≥ 85 cm in men and ≥ 90 cm in women (see e.g. investigating committee for the diagnosis of metabolic syndrome in Japan).

The term **"euglycemia"** is defined as the condition in which a subject has a fasting blood glucose concentration within the normal range, greater than 70 mg/dL (3.89
mmol/L) and less than 100 mg/dL (5.6 mmol/L). The word "fasting" has the usual meaning as a medical term.

The term **"hyperglycemia"** is defined as the condition in which a subject has a fasting blood glucose concentration above the normal range, greater than 100 mg/dL (5.6 mmol/L). The word "fasting" has the usual meaning as a medical term.

The term **"hypoglycemia"** is defined as the condition in which a subject has a blood glucose concentration below the normal range, in particular below 70 mg/dL (3.89 mmol/L) or even below 60 mg/dl.

The term **"postprandial hyperglycemia"** is defined as the condition in which a subject has a 2 hour postprandial blood glucose or serum glucose concentration greater than 200 mg/dL (11.1 mmol/L).

The term **"impaired fasting blood glucose"** or **"IFG"** is defined as the condition in which a subject has a fasting blood glucose concentration or fasting serum glucose concentration in a range from 100 to 125 mg/dl (i.e. from 5.6 to 6.9 mmol/l), in particular greater than 110 mg/dL and less than 126 mg/dl (7.00 mmol/L). A subject with "normal fasting glucose" has a fasting glucose concentration lower than 100 mg/dl, i.e. lower than 5.6 mmol/l.

The term **"impaired glucose tolerance"** or **"IGT"** is defined as the condition in which a subject has a 2 hour postprandial blood glucose or serum glucose concentration greater than 140 mg/dl (7.8 mmol/L) and less than 200 mg/dL (11.11 mmol/L). The abnormal glucose tolerance, i.e. the 2 hour postprandial blood glucose or serum glucose concentration can be measured as the blood sugar level in mg of glucose per dL of plasma 2 hours after taking 75 g of glucose after a fast. A subject with "normal glucose tolerance" has a 2 hour postprandial blood glucose or serum glucose concentration smaller than 140 mg/dl (7.8 mmol/L).

The term **"hyperinsulinemia"** is defined as the condition in which a subject with insulin resistance, with or without euglycemia, has a fasting or postprandial serum or plasma insulin concentration elevated above that of normal, lean individuals without insulin resistance, having a waist-to-hip ratio < 1.0 (for men) or < 0.8 (for women).

The terms **"insulin-sensitizing",** "insulin resistance-improving" or "insulin resistance-lowering" are synonymous and used interchangeably.

The term **"insulin resistance"** is defined as a state in which circulating insulin levels in excess of the normal response to a glucose load are required to maintain the euglycemic state (Ford ES, et al. JAMA. (2002) 287:356-9). A method of determining insulin resistance is the euglycaemic-hyperinsulinaemic clamp test. The ratio of insulin to glucose is determined within the scope of a combined insulin-glucose infusion technique. There is found to be insulin resistance

if the glucose absorption is below the 25th percentile of the background population investigated (WHO definition). Rather less laborious than the clamp test are so called minimal models in which, during an intravenous glucose tolerance test, the insulin and glucose concentrations in the blood are measured at fixed time intervals and from these the insulin resistance is calculated. With this method, it is not possible to distinguish between hepatic and peripheral insulin resistance.

Furthermore, insulin resistance, the response of a patient with insulin resistance to therapy, insulin sensitivity and hyperinsulinemia may be quantified by assessing the "homeostasis model assessment to insulin resistance (HOMA-IR)" score, a reliable indicator of insulin resistance (Katsuki A, et al. Diabetes Care 2001; 24: 362-5). Further reference is made to methods for the determination of the HOMA-index for insulin sensitivity (Matthews et al., Diabetologia 1985, 28: 412-19), of the ratio of intact proinsulin to insulin (Forst et al., Diabetes 2003, 52(Suppl.1): A459) and to an euglycemic clamp study. In addition, plasma adiponectin levels can be monitored as a potential surrogate of insulin sensitivity. The estimate of insulin resistance by the homeostasis assessment model (HOMA)-IR score is calculated with the formula (Galvin P, et al. Diabet Med 1992;9:921-8):

$$\text{HOMA-IR} = [\text{fasting serum insulin } (\mu U/mL)] \times [\text{fasting plasma glucose}(mmol/L)/22.5]$$

As a rule, other parameters are used in everyday clinical practice to assess insulin resistance. Preferably, the patient's triglyceride concentration is used, for example, as increased triglyceride levels correlate significantly with the presence of insulin resistance.

Patients with a predisposition for the development of IGT or IFG or type 2 diabetes are those having euglycemia with hyperinsulinemia and are by definition, insulin resistant. A typical patient with insulin resistance is usually overweight or obese, but this is not always the case. If insulin resistance can be detected, this is a particularly strong indication of the presence of pre-diabetes. Thus, it may be that in order to maintain glucose homoeostasis a person have e.g. 2-3 times as high endogenous insulin production as a healthy person, without this resulting in any clinical symptoms.

The methods to investigate the **function of pancreatic beta-cells** are similar to the above methods with regard to insulin sensitivity, hyperinsulinemia or insulin resistance: An improvement of beta-cell function can be measured for example by determining a HOMA-index for beta-cell function (Matthews et al., Diabetologia 1985, 28: 412-19), the ratio of intact proinsulin to insulin (Forst et al., Diabetes 2003, 52(Suppl.1): A459), the insulin/C-peptide secretion after an oral glucose tolerance test or a meal tolerance test, or by employing a hyperglycemic clamp study and/or minimal modeling after a frequently sampled intravenous glucose tolerance test (Stumvoll et al., Eur J Clin Invest 2001, 31: 380-81).

**"Pre-diabetes"** is a general term that refers to an intermediate stage between normal glucose tolerance (NGT) and overt type 2 diabetes mellitus (T2DM), also referred to as intermediate hyperglycaemia. As such, it represents 3 groups of individuals, those with impaired glucose tolerance (IGT) alone, those with impaired fasting glucose (IFG) alone or those with both IGT and IFG. IGT and IFG usually have distinct pathophysiologic etiologies, however also a mixed condition with features of both can exist in patients. Therefore in the context of the present invention a patient being diagnosed of having "pre-diabetes" is an individual with diagnosed IGT or diagnosed IFG or diagnosed with both IGT and IFG. Following the definition according to the American Diabetes Association (ADA) and in the context of the present invention a patient being diagnosed of having "pre-diabetes" is an individual with:

a) a fasting plasma glucose (FPG) concentration <100 mg/dL [1 mg/dL = 0.05555 mmol/L] and a 2-hour plasma glucose (PG) concentration, measured by a 75-g oral glucose tolerance test (OGTT), ranging between ≥140 mg/dL and <200 mg/dL (i.e., IGT); or
b) a fasting plasma glucose (FPG) concentration between ≥100 mg/dL and <126 mg/dL and a 2-hour plasma glucose (PG) concentration, measured by a 75-g oral glucose tolerance test (OGTT) of <140 mg/dL (i.e., IFG); or
c) a fasting plasma glucose (FPG) concentration between ≥100 mg/dL and <126 mg/dL and a 2-hour plasma glucose (PG) concentration, measured by a 75-g oral glucose tolerance test (OGTT), ranging between ≥140 mg/dL and <200 mg/dL (i.e., both IGT and IFG).

Patients with "pre-diabetes" are individuals being pre-disposed to the development of type 2 diabetes. Pre-diabetes extends the definition of IGT to include individuals with a fasting blood glucose within the high normal range ≥ 100 mg/dL (J. B. Meigs, et al. Diabetes 2003; 52:1475-1484). The scientific and medical basis for identifying pre-diabetes as a serious health threat is laid out in a Position Statement entitled "The Prevention or Delay of Type 2 Diabetes" issued jointly by the American Diabetes Association and the National Institute of Diabetes and Digestive and Kidney Diseases (Diabetes Care 2002; 25:742-749).

The term **"type 1 diabetes"** is defined as the condition in which a subject has, in the presence of autoimmunity towards the pancreatic beta-cell or insulin, a fasting blood glucose or serum glucose concentration greater than 125 mg/dL (6.94 mmol/L). If a glucose tolerance test is carried out, the blood sugar level of a diabetic will be in excess of

200 mg of glucose per dL (11.1 mmol/l) of plasma 2 hours after 75 g of glucose have been taken on an empty stomach, in the presence of autoimmunity towards the pancreatic beta cell or insulin. In a glucose tolerance test, 75 g of glucose are administered orally to the patient being tested after 10-12 hours of fasting and the blood sugar level is recorded immediately before taking the glucose and 1 and 2 hours after taking it. The presence of autoimmunity towards the pancreatic beta-cell may be observed by detection of circulating islet cell autoantibodies ["type 1A diabetes mellitus"], i.e., at least one of: GAD65 [glutamic acid decarboxylase-65], ICA [islet-cell cytoplasm], IA-2 [intracytoplasmatic domain of the tyrosine phosphatase-like protein IA-2], ZnT8 [zinc-transporter-8] or anti-insulin; or other signs of autoimmunity without the presence of typical circulating autoantibodies [type 1B diabetes], i.e. as detected through pancreatic biopsy or imaging). Typically, a genetic predisposition is present (e.g. HLA, *INS* VNTR and *PTPN22),* but this is not always the case.

The term **"type 2 diabetes"** is defined as the condition in which a subject has a fasting blood glucose or serum glucose concentration greater than 125 mg/dL (6.94 mmol/L). The measurement of blood glucose values is a standard procedure in routine medical analysis. If a glucose tolerance test is carried out, the blood sugar level of a diabetic will be in excess of 200 mg of glucose per dL (11.1 mmol/l) of plasma 2 hours after 75 g of glucose have been taken on an emptystomach. In a glucose tolerance test, 75 g of glucose are administered orally to the patient being tested after 10-12 hours of fasting and the blood sugar level is recorded immediately before taking the glucose and 1 and 2 hours after taking it. In a healthy subject, the blood sugar level before taking the glucose will be between 60 and 110 mg per dL of plasma, less than 200 mg per dL 1 hour after taking the glucose and less than 140 mg per dL after 2 hours. If after 2 hours the value is between 140 and 200 mg, this is regarded as abnormal glucose tolerance.

The term **"late stage type 2 diabetes mellitus"** includes patients with a secondary drug failure, indication for insulin therapy and progression to micro- and macrovascular complications e.g. diabetic nephropathy, or coronary heart disease (CHD).

The term **"HbA1c"** refers to the product of a non-enzymatic glycation of the haemoglobin B chain. Its determination is well known to one skilled in the art. In monitoring the treatment of diabetes mellitus the HbA1c value is of exceptional importance. As its production depends essentially on the blood sugar level and the life of the erythrocytes, the HbA1c in the sense of a "blood sugar memory" reflects the average blood sugar levels of the preceding 8-12 weeks. Diabetic patients whose HbA1c value is consistently well adjusted by intensive diabetes treatment (i.e. < 6.5 % of the total haemoglobin in the sample), are significantly better protected against diabetic microangiopathy. For example, metformin on its own achieves an average improvement in the HbA1c value in the diabetic of the order of 1.0 - 1.5 %. This reduction of the HbA1C value is not sufficient in all diabetics to achieve the desired target range of < 6.5 % and preferably < 6 % HbA1c.

The term **"insufficient glycemic control"** or "inadequate glycemic control" in the scope of the present invention means a condition wherein patients show HbA1c values above 6.5 %, in particular above 7.0 %, even more preferably above 7.5 %, especially above 8 %.

The **"metabolic syndrome",** also called "syndrome X" (when used in the context of a metabolic disorder), also called the "dysmetabolic syndrome" is a syndrome complex with the cardinal feature being insulin resistance (Laaksonen DE, et al. Am J Epidemiol 2002;156:1070-7). According to the ATP III/NCEP guidelines (Executive Summary of the Third Report of the National Cholesterol Education Program (NCEP) Expert Panel on Detection, Evaluation, and Treatment of High Blood Cholesterol in Adults (Adult Treatment Panel III) JAMA: Journal of the American Medical Association (2001) 285:2486-2497), diagnosis of the metabolic syndrome is made when three or more of the following risk factors are present:

1. Abdominal obesity, defined as waist circumference > 40 inches or 102 cm in men, and > 35 inches or 94 cm in women; or with regard to a Japanese ethnicity or Japanese patients defined as waist circumference ≥ 85 cm in men and ≥ 90 cm in women;
2. Triglycerides: ≥ 150 mg/dL
3. HDL-cholesterol < 40 mg/dL in men
4. Blood pressure ≥ 130/85 mm Hg (SBP ≥ 130 or DBP ≥ 85)
5. Fasting blood glucose ≥ 100 mg/dL

The NCEP definitions have been validated (Laaksonen DE, et al. Am J Epidemiol. (2002) 156:1070-7). Triglycerides and HDL cholesterol in the blood can also be determined by standard methods in medical analysis and are described for example in Thomas L (Editor): "Labor und Diagnose", TH-Books Verlagsgesellschaft mbH, Frankfurt/Main, 2000.

According to a commonly used definition, **hypertension** is diagnosed if the systolic blood pressure (SBP) exceeds a value of 140 mm Hg and diastolic blood pressure (DBP) exceeds a value of 90 mm Hg. If a patient is suffering from manifest diabetes it is currently recommended that the systolic blood pressure be reduced to a level below 130 mm Hg and the diastolic blood pressure be lowered to below 80 mm Hg.

The term **"gestational diabetes"** (diabetes of pregnancy) denotes a form of the diabetes which develops during pregnancy and usually ceases again immediately after the birth. Gestational diabetes is diagnosed by a screening test which is carried out between the 24th and 28th weeks of pregnancy. It is usually a simple test in which the blood sugar level is measured one hour after the administration of 50 g of glucose solution. If this 1 h level is above 140 mg/dl, gestational diabetes is suspected. Final confirmation may be obtained by a standard glucose tolerance test, for example with 75 g of glucose.

The term **"SGLT2 inhibitor"** in the scope of the present invention relates to a compound, in particular to a glucopyranosyl-derivative, i.e. compound having a glucopyranosyl-moiety, which shows an inhibitory effect on the sodium-glucose transporter 2 (SGLT2), in particular the human SGLT2. The inhibitory effect on hSGLT2 measured as $IC_{50}$ is preferably below 1000 nM, even more preferably below 100 nM, most preferably below 50 nM. $IC_{50}$ values of SGLT2 inhibitors are usually above 0.01 nM, or even equal to or above 0.1 nM. The inhibitory effect on hSGLT2 can be determined by methods known in the literature, in particular as described in the application WO 2005/092877 or WO 2007/093610 (pages 23/24). The term "SGLT2 inhibitor" also comprises any pharmaceutically acceptable salts thereof, hydrates and solvates thereof, including the respective crystalline forms.

The terms **"treatment"** and "treating" comprise therapeutic treatment of patients having already developed a condition, in particular in manifest form. Therapeutic treatment may be symptomatic treatment in order to relieve the symptoms of the specific indication or causal treatment in order to reverse or partially reverse the conditions of the indication or to stop or slow down progression of the disease. Thus the compositions and methods of the present invention may be used for instance as therapeutic treatment over a period of time as well as for chronic therapy.

The terms **"prophylactically treating",** "preventivally treating" and "preventing" are used interchangeably and comprise a treatment of patients at risk to develop a condition mentioned hereinbefore, thus reducing said risk.

**Brief Description of the Figures**

Figure 1A: Oral glucose tolerance test for selected neuroleptic agents.
Figure 1B: Glucose Area Under the Curve (AUC) for selected neuroleptic agents.
Figure 2A: Oral glucose tolerance test for olanzapine in combination with selected SGLT-2 inhibitors.
Figure 2B: Glucose AUC for olanzapine in combination with selected SGLT-2 inhibitors.
Figure 3A: Oral glucose tolerance test for clozapine in combination with selected SGLT-2 inhibitors.
Figure 3B: Glucose AUC for clozapine in combination with selected SGLT-2 inhibitors.
Figure 4A: Oral glucose tolerance test for haloperidone in combination with selected SGLT-2 inhibitors.
Figure 4B: Glucose AUC for haloperidone in combination with selected SGLT-2 inhibitors.

**Detailed Description**

The aspects according to the present invention, in particular the methods and uses, refer to SGLT2 inhibitors and neuroleptic agents.

Renal filtration and reuptake of glucose contributes, among other mechanisms, to the steady state plasma glucose concentration and can therefore serve as an antidiabetic target. Reuptake of filtered glucose across epithelial cells of the kidney proceeds via sodium-dependent glucose cotransporters (SGLTs) located in the brush-border membranes in the tubuli along the sodium gradient. There are at least 3 SGLT isoforms that differ in their expression pattern as well as in their physico-chemical properties. SGLT2 is exclusively expressed in the kidney, whereas SGLT1 is expressed additionally in other tissues like intestine, colon, skeletal and cardiac muscle. Under normoglycemia, glucose is completely reabsorbed by SGLTs in the kidney, whereas the reuptake capacity of the kidney is saturated at glucose concentrations higher than 10mM, resulting in glucosuria (hence the notion "diabetes mellitus"). This threshold concentration can be decreased by SGLT2-inhibition. It has been shown in experiments with the SGLT inhibitor phlorizin that SGLT-inhibition will partially inhibit the reuptake of glucose from the glomerular filtrate into the blood leading to glucosuria and subsequently to a decrease in blood glucose concentration.

Compounds of the formula (I)

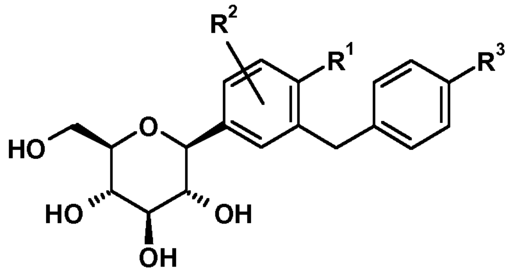

I

wherein $R^1$ denotes Cl, methyl or cyano; $R^2$ denotes H, methyl, methoxy or hydroxy and $R^3$ denotes ethyl, cyclopropyl, ethynyl, ethoxy, (*R*)-tetrahydrofuran-3-yloxy or (*S*)-tetrahydrofuran-3-yloxy;
and methods of their synthesis are described for example in the following patent applications: WO 2005/092877, WO 2006/117360, WO 2006/117359, WO 2006/120208, WO 2006/064033, WO 2007/031548, WO 2007/093610, WO 2008/020011, WO 2008/055870, WO 2011/039107, and WO 2011/039108.

A preferred glucopyranosyl-substituted benzene derivative is the compound (I.9):

| | |
|---|---|
| (I.9) | 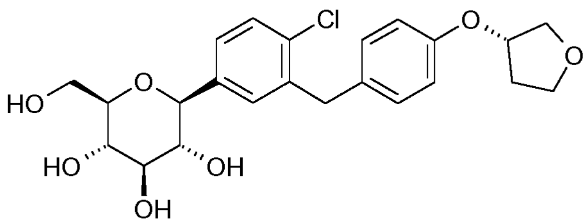<br>1-chloro-4-(β-D-glucopyranos-1-yl)-2-[4-((*S*)-tetrahydrofuran-3-yloxy)-benzyl]-benzene. |

According to an embodiment of the present invention, the SGLT2 inhibitor is the compound (1.9), also called empagliflozin.

According to another embodiment of the present invention, SGLT2 inhibitor is selected from the group consisting of dapagliflozin and canagliflozin.

According to this invention, it is to be understood that the definitions of the above listed SGLT2 inhibitors, including the glucopyranosyl-substituted benzene derivative of the formula (1.9), also comprise their hydrates, solvates and polymorphic forms thereof, and prodrugs thereof. With regard to the preferred compound (I.9) an advantageous crystalline form is described in the international patent application WO 2006/117359 and WO 2011/039107. These crystalline forms possess good solubility properties which enable a good bioavailability of the SGLT2 inhibitor. Furthermore, the crystalline forms are physicochemically stable and thus provide a good shelf-life stability of the pharmaceutical composition.

A preferred crystalline form (I.9X) of the compound (I.9) can be characterized by an X-ray powder diffraction pattern that comprises peaks at 18.84, 20.36 and 25.21 degrees 2Θ (±0.1 degrees 2Θ), wherein said X-ray powder diffraction pattern (XRPD) is made using $CuK_{\alpha1}$ radiation.

In particular said X-ray powder diffraction pattern comprises peaks at 14.69, 18.84, 19.16, 19.50, 20.36 and 25.21 degrees 2Θ (±0.1 degrees 2Θ), wherein said X-ray powder diffraction pattern is made using $CuK_{\alpha1}$ radiation.

In particular said X-ray powder diffraction pattern comprises peaks at 14.69, 17.95, 18.43, 18.84, 19.16, 19.50, 20.36, 22.71, 23.44, 24.81, 25.21 and 25.65 degrees 2Θ (±0.1 degrees 2Θ), wherein said X-ray powder diffraction pattern is made using $CuK_{\alpha1}$ radiation.

More specifically, the crystalline form (I.9X) is characterized by an X-ray powder diffraction pattern, made using $CuK_{\alpha1}$ radiation, which comprises peaks at degrees 2Θ (±0.1 degrees 2Θ) as contained in Table 1. Particularly characteristic are peaks with a relative intensity $I/I_0$ above 20.

**Table** 1: X-ray powder diffraction pattern of the crystalline form (I.9X) (only peaks up to 30° in 2 Θ are listed):

| **2 Θ [°]** | **d-value [Å]** | **Intensity $I/I_0$ [%]** |
|---|---|---|
| 4.46 | 19.80 | 8 |
| 9.83 | 8.99 | 4 |

(continued)

| **2 Θ [°]** | **d-value [Å]** | **Intensity $I/I_0$ [%]** |
|---|---|---|
| 11.68 | 7.57 | 4 |
| 13.35 | 6.63 | 14 |
| 14.69 | 6.03 | 42 |
| 15.73 | 5.63 | 16 |
| 16.20 | 5.47 | 8 |
| 17.95 | 4.94 | 30 |
| 18.31 | 4.84 | 22 |
| 18.43 | 4.81 | 23 |
| 18.84 | 4.71 | 100 |
| 19.16 | 4.63 | 42 |
| 19.50 | 4.55 | 31 |
| 20.36 | 4.36 | 74 |
| 20.55 | 4.32 | 13 |
| 21.18 | 4.19 | 11 |
| 21.46 | 4.14 | 13 |
| 22.09 | 4.02 | 19 |
| 22.22 | 4.00 | 4 |
| 22.71 | 3.91 | 28 |
| 23.44 | 3.79 | 27 |
| 23.72 | 3.75 | 3 |
| 24.09 | 3.69 | 3 |
| 24.33 | 3.66 | 7 |
| 24.81 | 3.59 | 24 |
| 25.21 | 3.53 | 46 |
| 25.65 | 3.47 | 23 |
| 26.40 | 3.37 | 2 |
| 26.85 | 3.32 | 8 |
| 27.26 | 3.27 | 17 |
| 27.89 | 3.20 | 2 |
| 28.24 | 3.16 | 3 |
| 29.01 | 3.08 | 4 |
| 29.41 | 3.03 | 18 |

Even more specifically, the crystalline form (I.9X) is characterized by an X-ray powder diffraction pattern, made using $CuK_{\alpha1}$ radiation, which comprises peaks at degrees 2Θ (±0.1 degrees 2Θ) as shown in Figure 1 of WO 2006/117359.

Furthermore, the crystalline form (I.9X) is characterized by a melting point of about 149°C ± 5°C (determined via DSC; evaluated as onset-temperature; heating rate 10 K/min). The obtained DSC curve is shown in Figure 2 of WO 2006/117359.

The X-ray powder diffraction patterns are recorded, within the scope of the present invention, using a STOE - STADI P-diffractometer in transmission mode fitted with a location-sensitive detector □(OED) and a Cu-anode as X-ray source (CuKα1 radiation, □ λ = 1,54056 Å, 40kV, 40mA). In the Table 1 above the values "2Θ[°]" denote the angle of diffraction in degrees and the values "d[Å]" denote the specified distances in Å between the lattice planes. The intensity shown in the Figure 1 of WO 2006/117359 is given in units of cps (counts per second).

In order to allow for experimental error, the above described 2Θ values should be considered accurate to ± 0.1 degrees 2Θ, in particular ± 0.05 degrees 2Θ. That is to say, when assessing whether a given sample of crystals of the compound (1.9) is the crystalline form in accordance with the invention, a 2Θ value which is experimentally observed for the sample should be considered identical with a characteristic value described above if it falls within ± 0.1 degrees 2Θ of the characteristic value, in particular if it falls within ± 0.05 degrees 2Θ of the characteristic value.

The melting point is determined by DSC (Differential Scanning Calorimetry) using a DSC 821 (Mettler Toledo).

In one embodiment, a pharmaceutical composition or dosage form according to the present invention comprises the compound (1.9), wherein at least 50 % by weight of the compound (1.9) is in the form of its crystalline form (I.9X) as defined hereinbefore. Preferably in said composition or dosage form at least 80 % by weight, more preferably at least 90 % by weight of the compound (1.9) is in the form of its crystalline form (I.9X) as defined hereinbefore.

The term "dapagliflozin" as employed herein refers to dapagliflozin, including hydrates and solvates thereof, and crystalline forms thereof. The compound and methods of its synthesis are described in WO 03/099836 for example. Preferred hydrates, solvates and crystalline forms are described in the patent applications WO 2008/116179 and WO 2008/002824 for example.

The term "canagliflozin" as employed herein refers to canagliflozin, including hydrates and solvates thereof, and crystalline forms thereof and has the following structure:

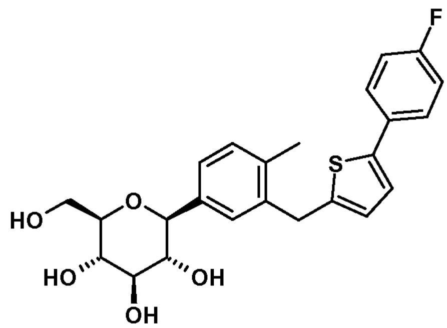

The compound and methods of its synthesis are described in WO 2005/012326 and WO 2009/035969 for example. Preferred hydrates, solvates and crystalline forms are described in the patent applications WO 2008/069327 for example.

Neuroleptic agents that are useful in the present invention in combination with a SGLT-2 inhibitor include, but are not limited to typical and atypical antipsychotic drugs, including phenothiazines, further divided into the aliphatics, piperidines, and piperazines, thioxanthenes (e.g., droperidol), butyrophenones (e.g., haloperidol), dibenzoxazepines (e.g., loxapine), dihydroindolones (e.g., molindone), diphenylbutylpiperidines (e.g., pimozide), and typical antipsychotic drugs, including benzisoxazoles (e.g., risperidone), olanzapine, quetiapine, osanetant and ziprasidone.

Accordingly, suitable neuroleptic agents for use in combination with a SGLT-2 inhibitor according to the present invention include butyrophenones, such as haloperidol, pimozide, and droperidol. Suitable examples of phenothiazines include chlorpromazine, mesoridazine, trifluoperazine, perphenazine, fluphenazine, thiflupromazine, prochlorperazine, thioridazine and acetophenazine. Suitable examples of thioxanthenes include thiothixene and chlorprothixene.
Suitable neuroleptic agents for use in combination with a SGLT-2 inhibitor according to the present invention also include thienobenzodiazepines; dibenzodiazepines; benzisoxazoles; dibenzothiazepines; imidazolidinones; benzisothiazolyl-piperazines.
Suitable neuroleptic agents for use in combination with a SGLT-2 inhibitor according to the present invention also include triazines such as lamotrigine; dibenzoxazepines, such as loxapine; dihydroindolones, such as molindone; aripiprazole.
Suitable neuroleptic agents for use in combination with a SGLT-2 inhibitor according to the present invention also include dibenzazepines such as clozapine.
Other neuroleptic agents for use in combination with a SGLT-2 inhibitor according to the present invention also include sulpiride.
Particularly suitable neuroleptic agents for use in the invention are neuroleptic agents selected from the group G2a selected from olanzapine, risperidone, quetiapine, amisulpiride, aripiprazole, haloperidol, clozapine, ziprasidone, zotepine, paliperidone and osanetant. Particularly suitable neuroleptic agents for use in the invention are olanzapine, clozapine, risperidone and quetiapine.

Haloperidol has the following structure:

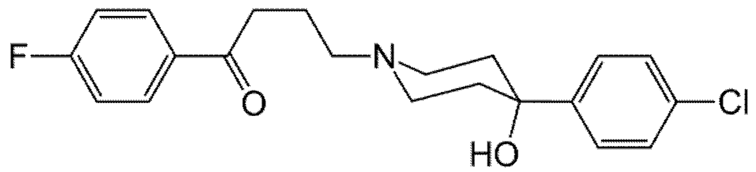

Clozapin has the following structure:

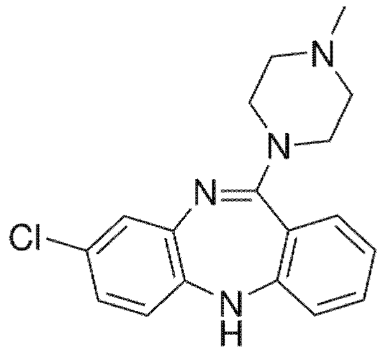

Olanzapine has the following structure:

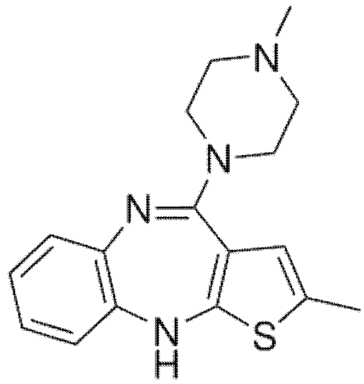

Risperidon has the following structure:

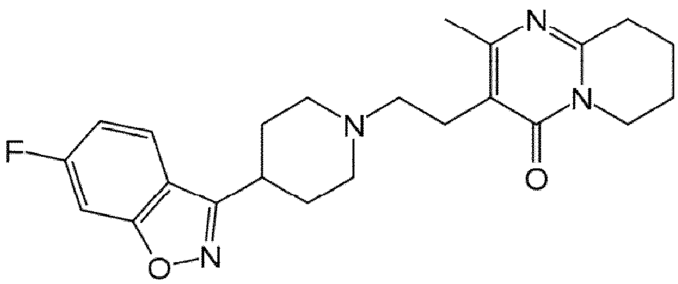

Quetiapin has the following structure:

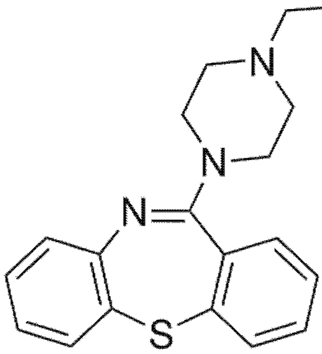

Amisulpirid has the following structure:

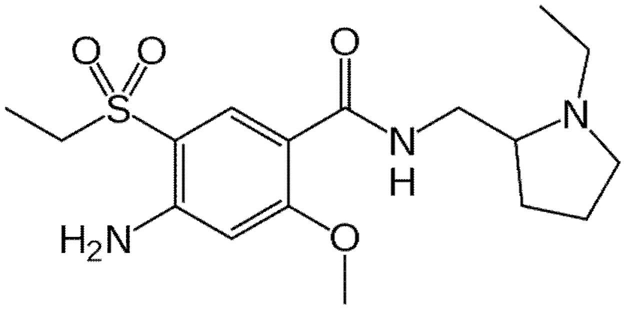

Sulpirid has the following structure:

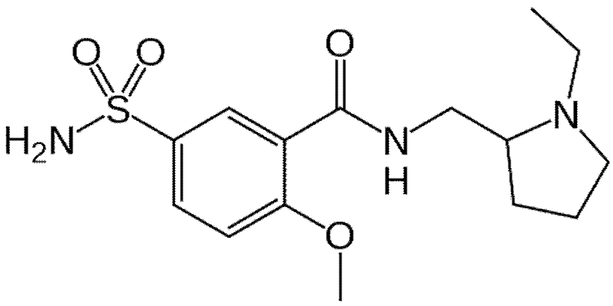

Additional suitable neuroleptic agents for use in combination with a SGLT-2 inhibitor according to the present invention also include neuroleptic agents selected form the group G2b consisting of asenapine, blonanserin, iloperidone, lurasidone, mosapramine, paliperidone, pericyazine, perospirone, promazine and zuclopenthixol.

Additional suitable neuroleptic agents for use in combination with a SGLT-2 inhibitor according to the present invention also include combinations of two or more of the above neuroleptic agents or combinations including one or more of the above neuroleptic agents with one or more additional compounds, for example olanzapine and fluoxetine or perphenazine and amitriyptyline.

The chemical names of selected compounds for use in the context of the present invention are shown below (group G2):

| INN | IUPAC |
|---|---|
| Amisulpiride | 4-amino-*N*-[(1-ethylpyrrolidin-2-yl)methyl]-5-ethylsulfonyl-2-methoxy-benzamide |
| Aripiprazole | 7-{4-[4-(2,3-dichlorophenyl)piperazin-1-yl]butoxy}-3,4-dihydroquinolin-2(*H*)-one-2,6-diazabicyclo[4.4.0]deca-1,3-dien-5-one |
| Asenapine | (3a*S*,12b*S*)-5-Chloro-2,3,3a,12b-tetrahydro-2-methyl-1H-dibenz[2,3:6,7]oxepino[4,5-c]pyrrole |
| Blonanserin | 2-(4-ethylpiperazin-1-yl)-4-(4-fluorophenyl)-5,6,7,8,9,10-hexahydrocycloocta[b]pyridine |

(continued)

| INN | IUPAC |
|---|---|
| Chlorpromazine | 3-(2-chloro-10*H*-phenothiazin-10-yl)-*N*,*N*-dimethyl-propan-1-amine |
| Clozapine | 8-chloro-11-(4-methylpiperazin-1-yl)-5H-dibenzo[b,e][1,4]diazepine |
| Doperidol | 1-{1-[4-(4-fluorophenyl)-4-oxobutyl]-1,2,5,6-tetrahydropyridin-4-yl]-1,3-dihydro-2*H*-benzimidazol-2-one |
| Fluphenazine | 2-[4-[3-[2-(trifluoromethyl)-10*H*-phenothiazin-10-yl]propyl]piperazin-1-yl]ethanol |
| Haloperidol | 4-[4-(4-chlorophenyl)-4-hydroxy-1-piperidyl]-1-(4-fluorophenyl)-butan-1-one |
| Iloperidone | 1-[4-[3-[4-(6-fluoro-1,2-benzisoxazol-3-yl)-1-piperidinyl]propoxy]-3-methoxyphenyl]ethanone |
| Lurasidone | (3a*R*,4*S*,7*R*,7a*S*)-2-[((1 *R*,2*R*)-2-{[4-(1,2-benzisothiazol-3-yl)-piperazin-1-yl]methyl}cyclohexyl) methyl]hexahydro-1 *H*-4,7-methanisoindol-1,3-dione |
| Mosapramine | 1'-[3-(3-chloro-10,11-dihydro-5*H*-dibenzo[*b*,*f*]azepin-5-yl)propyl]hexahydro-2*H*-spiro[imidazo [1,2-*a*]pyridine-3,4'-piperidin]-2-one |
| Olanzapine | 2-methyl-4-(4-methyl-1-piperazinyl)-10*H*-thieno[2,3-*b*][1,5]benzodiazepine |
| Osanetant | *N*-(1-{3-[(3*R*)-1 -benzoyl-3-(3,4-dichlorophenyl)piperidin-3-yl]propyl}-4-phenylpiperidin-4-yl]-*N*-methylacetamide |
| Paliperidone | (*RS*)-3-[2-[4-(6-fluorobenzo[*d*]isoxazol-3-yl)-1-piperidyl]ethyl]-7-hydroxy-4-methyl-1,5-diazabicyclo[4.4.0]deca-3,5-dien-2-one |
| Pericyazine | 10-[3-(4-hydroxypiperidin-1-yl)propyl]-10*H*-phenothiazine-2-carbonitrile |
| Perospirone | (3a*R*,7a*S*)-2-{4-[4-(1,2-benzisothiazol-3-yl)piperazin-1-yl]butyl}hexahydro-1*H*-isoindole-1,3 (2*H*)-dione |
| Perphenazine | 2-[4-[3-(2-chloro-10H-phenothiazin-10-yl) propyl]piperazin-1-yl]ethanol |
| Pimozide | 1-[1-[4,4-bis(4-fluorophenyl)butyl]-4-piperidinyl]-1,3-dihydro-2H-benzimidazole-2-one |
| Prochlorperazine | 2-chloro-10-[3-(4-methyl-1-piperazinyl)propyl]-10H-phenothiazine |
| Promazine | N,N-dimethyl-3-(10H-phenothiazin-10-yl)-propan-1-amine |
| Quetiapine | 2-(2-(4-dibenzo[*b*,*f*][1,4]thiazepine-11-yl-1-piperazinyl)ethoxy)ethanol |
| Risperidone | 4-[2-[4-(6-fluorobenzo[*d*]isoxazol-3-yl)-1-piperidyl]ethyl]-3-methyl-2,6-diazabicyclo[4.4.0]deca-1,3-dien-5-one |
| Sulpiride | (±)-5-(aminosulfonyl)-*N*-[(1-ethylpyrrolid in-2-yl)methyl]-2-methoxybenzamide |
| Thioridazine | 10-{2-[(*RS*)-1-Methylpiperidin-2-yl]ethyl}-2-methylsulfanylphenothiazine |
| Thiothixene | (9*Z*)-*N*,*N*-dimethyl-9-[3-(4-methylpiperazin-1-yl)propylidene]-9*H*-thioxanthene-2-sulfonamide |
| Trifluoperazine | 10-[3-(4-methylpiperazin-1-yl)propyl]-2-(trifluoromethyl)-10H-phenothiazine |
| Ziprasidone | 5-[2-[4-(1,2-benzisothiazol-3-yl)-1-piperazinyl]ethyl]-6-chloro-1,3-dihydro-2H-indol-2-one |
| Zotepine | 2-[(8-chlorodibenzo(*b*,*f*)thiepin-10-yl)oxy]-*N*,*N*-dimethylethanamine |
| Zuclopenthixol | *cis*-(Z)-2-(4-(3-(2-chloro-9H-thioxanthen-9-ylidene)propyl)piperazin-1-yl)ethanol |

It will be appreciated that the neuroleptic agents when used in combination with an SGLT-2 inhibitor may be in the form of a pharmaceutically acceptable salt, for example, chlorpromazine hydrochloride, mesoridazine besylate, thioridazine hydrochloride, acetophenazine maleate, fluphenazine hydrochloride, flurphenazine enathate, fluphenazine decanoate, trifluoperazine hydrochloride, thiothixene hydrochloride, haloperidol decanoate, loxapine succinate and molindone hydrochloride. Perphenazine, chlorprothixene, clozapine, haloperidol, pimozide and risperidone are commonly used in a non-salt form.

Unless otherwise noted, according to this invention it is to be understood that the definitions of the active agents (including the SGLT2 inhibitors and neuroleptic agents) mentioned hereinbefore and hereinafter may also contemplate their pharmaceutically acceptable salts, and prodrugs, hydrates, solvates and polymorphic forms thereof. Particularly the terms

of the therapeutic agents given herein refer to the respective active drugs. With respect to salts, hydrates and polymorphic forms thereof, particular reference is made to those which are referred to herein.

In a further embodiment, the compositions and uses according to this invention relate to combinations wherein the SGLT2 inhibitor is selected according to the invention and the neuroleptic agent is selected from the group G2.

In a further embodiment, the compositions and uses according to this invention relate to combinations wherein the SGLT2 inhibitor is selected according to the invention and the neuroleptic agent is selected from the group G2a.

In a further embodiment, the compositions and uses according to this invention relate to combinations wherein the SGLT2 inhibitor is selected according to the invention and the neuroleptic agent is selected from the group G2b.

In a further embodiment, the compositions and uses according to this invention relate to combinations wherein the SGLT2 inhibitor is empagliflozin and the neuroleptic agent is selected from the group G2.

In a further embodiment, the compositions and uses according to this invention relate to combinations wherein the SGLT2 inhibitor is empagliflozin and the neuroleptic agent is selected from the group G2a.

In a further embodiment, the compositions and uses according to this invention relate to combinations wherein the SGLT2 inhibitor is empagliflozin and the neuroleptic agent is selected from the group G2b.

In a particular embodiment, the compositions and uses according to this invention relate to combinations wherein the SGLT2 inhibitor is the compound of the formula (I.9), also called empagliflozin.

In a further aspect, the compositions and uses according to this invention relate to combinations wherein the SGLT2 inhibitor and the neuroleptic agent are as follows:

| SGLT-2 inhibitor | Neuroleptic agent |
|---|---|
| Empagliflozin | Olanzapine |
| Empagliflozin | Clozapine |
| Empagliflozin | Risperidone |
| Empagliflozin | Quetiapine |
| Empagliflozin | Paliperidone |
| Empagliflozin | Aripiprazole |

In a further aspect, the compositions and uses according to this invention relate to combinations wherein the SGLT2 inhibitor and the neuroleptic agent are as follows:

| SGLT-2 inhibitor | Neuroleptic agent |
|---|---|
| Dapagliflozin | Olanzapine |
| Dapagliflozin | Clozapine |
| Dapagliflozin | Risperidone |
| Dapagliflozin | Quetiapine |
| Dapagliflozin | Paliperidone |
| Dapagliflozin | Aripiprazole |

In a further aspect, the compositions and uses according to this invention relate to combinations wherein the SGLT2 inhibitor and the neuroleptic agent are as follows:

| SGLT-2 inhibitor | Neuroleptic agent |
|---|---|
| Canagliflozin | Olanzapine |
| Canagliflozin | Clozapine |
| Canagliflozin | Risperidone |
| Canagliflozin | Quetiapine |
| Canagliflozin | Paliperidone |
| Canagliflozin | Aripiprazole |

Accordingly, in the context of the present invention, an SGLT-2 inhibitor according to the present invention can be useful to compensate the side effects resulting from the administration of a neuroleptic agent in a patient, in particular metabolic side effects. In one aspect, an SGLT-2 inhibitor according to the present invention can be useful to compensate the weight gain in a patient resulting from the administration of a neuroleptic agent to the patient. In another aspect, an SGLT-2 inhibitor according to the present invention can be useful to compensate hyperglycemia in a patient resulting from the administration of a neuroleptic agent to the patient. As described hereinbefore by the use according to this invention or the administration of a pharmaceutical composition according to this invention and in particular in view of the effect of the SGLT2 inhibitors therein, a reduction of weight gain due to the administration of neuroleptic agent may result, or no gain in weight or even a reduction in body weight may result. In some instances, a metabolic side effect of the treatment with certain neuroleptic agents may be an increase in blood pressure associated with an increase in body weight, for example an increase in systolic or diastolic blood pressure, or both. In these instances, an SGLT-2 inhibitor according to the present invention may be useful to compensate such increase in blood pressure (systolic or diastolic blood pressure, or both) in a patient resulting from the administration of a neuroleptic agent to the patient. Accordingly, in one aspect, the present disclosure provides a method for treating, for reducing, for preventing or for attenuating hypertension associated with weight gain in a patient treated for a psychotic disorder, said method comprising administering to said patient a SGLT2 inhibitor and a neuroleptic agent. In a further aspect, the present disclosure provides the use of a SGLT2 inhibitor for treating, for reducing, for preventing or for attenuating hypertension associated with weight gain in a patient treated with a neuroleptic agent.

In a further aspect, an SGLT-2 inhibitor according to the present invention can be useful to reduce or prevent discontinuation of treatment with a neuroleptic agent in a patient treated with such neuroleptic agent.

In the context of the present invention, a metabolic disorder includes type 2 diabetes mellitus, impaired glucose tolerance (IGT), impaired fasting blood glucose (IFG), hyperglycemia, postprandial hyperglycemia, overweight, obesity, metabolic syndrome, gestational diabetes and diabetes related to cystic fibrosis. A metabolic disorder in the context of the present invention also includes weight gain. A metabolic disorder in the context of the present invention also includes pre-diabetes. A metabolic disorder in the context of the present invention may be also hypertension associated with weight gain.

In a further aspect, a treatment or prophylaxis according to this invention is advantageously suitable in those patients in need of such treatment or prophylaxis, for example patients treated with a neuroleptic agent, who are diagnosed of one or more of the conditions selected from the group consisting of overweight and obesity, in particular class I obesity, class II obesity, class III obesity, visceral obesity and abdominal obesity. In addition, a treatment or prophylaxis according to this invention is advantageously suitable in those patients in which a weight increase is contraindicated.

When this invention refers to patients requiring treatment or prevention, it relates primarily to treatment and prevention in humans, but the methods and pharmaceutical compositions of the present invention may also be used accordingly in veterinary medicine in mammals. In the scope of this invention the term "patient" covers adult humans (age of 18 years or older), adolescent humans (age 10 to 17 years) and children (age 6-9 years).

In one aspect of the invention, a psychotic disorder is schizophrenia. In one aspect of the invention, a patient is a subject treated for a psychotic disorder, for example schizophrenia.

In one aspect of the invention, symptom or psychosis severity in subjects with schizophrenia is measured using a PANSS score (Positive and Negative Symdrom Scale). The PANSS score is well known in the art.

In one aspect of the invention, a patient in a combination, composition, method or use according to the present invention is a subject is treated for one of the following disorders:

- psychosis,
- acute and chronic psychosis,
- acute psychotic state,
- psychosis in major depression,
- agitation in schizophrenia or bipolar disorders,
- treatment-resistant schizophrenia,
- acute agitation in schizophrenia,
- delirium,
- delirium in AIDS.

In another aspect of the invention, a patient in a combination, composition, method or use according to the present invention is a subject is treated for depression. In a further aspect, a patient is a subject is treated for one of the following disorders:

- agitated depression,
- adjunct in major depression,

- dysthymia,
- bipolar disorders,
- manic phase of bipolar disorder,
- bipolar mania.

In another aspect of the invention, a patient in a combination, composition, method or use according to the present invention is a subject is treated for manic episodes associated with bipolar I disorder. In another aspect, a patient is a subject is treated for mixed episodes associated with bipolar I disorder. In one other aspect, a patient is a subject is treated for manic or mixed episodes associated with bipolar I disorder. In another aspect, a patient is a subject is treated for acute agitation associated with schizophrenia and bipolar I mania. In another aspect, a patient is a subject is treated for depressive episodes associated with bipolar I disorder.

In a further aspect of the invention, a patient in a combination, composition, method or use according to the present invention is a subject is treated for one of the following other mental states leading to mental disturbances or mental dysfunction:

- insomnia,
- pruritus,
- preanesthesia,
- suicidal behavior,
- anxiety,
- post-traumatic stress disorder (PTSD),
- autism,
- tension and anxiety linked to alcohol withdrawal,
- dysphoria of epileptic,
- severe anxiety.

According to an embodiment of the present invention, there is provided a SGLT2 inhibitor for use in a method for improving glycemic control in a patient treated with a neuroleptic agent who is diagnosed with impaired glucose tolerance (IGT), impaired fasting blood glucose (IFG) with insulin resistance, with metabolic syndrome and/or with type 1 diabetes mellitus or type 2 diabetes mellitus characterized in that a neuroleptic agent and an SGLT2 inhibitor as defined hereinbefore and hereinafter are administered, for example in combination or alternation or sequentially, to the patient.

Furthermore, the uses and the pharmaceutical composition, according to this invention are particularly suitable in the treatment of patients treated with a neuroleptic agent who are diagnosed having one or more of the following conditions

(a) obesity (including class I, II and/or III obesity), visceral obesity and/or abdominal obesity,
(b) triglyceride blood level ≥ 150 mg/dL,
(c) HDL-cholesterol blood level < 40 mg/dL in female patients and < 50 mg/dL in male patients,
(d) a systolic blood pressure ≥ 130 mm Hg and a diastolic blood pressure ≥ 85 mm Hg,
(e) a fasting blood glucose level ≥ 100 mg/dL.

It is assumed that patients treated with a neuroleptic agent and diagnosed with impaired glucose tolerance (IGT), impaired fasting blood glucose (IFG), with insulin resistance and/or with metabolic syndrome suffer from an increased risk of developing a cardiovascular disease, such as for example myocardial infarction, coronary heart disease, heart insufficiency, thromboembolic events. A glycemic control according to this invention may result in a reduction of the neuroleptic-induced side effects including cardiovascular risks. A method or pharmaceutical composition according to this invention can be particularly suitable in the long term treatment or prophylaxis of the diseases and/or conditions as described hereinbefore and hereinafter, in particular in the long term glycemic control in patients with type 2 diabetes mellitus being treated with a neuroleptic agent, such as a typical or atypical neuroleptic agent.

The term "long term" as used hereinbefore and hereinafter indicates a treatment of or administration in a patient within a period of time longer than 12 weeks, preferably longer than 25 weeks, even more preferably longer than 1 year.

Therefore, a particularly preferred embodiment of the present invention provides a SGLT2 inhibitor for use in a method for therapy, preferably oral therapy, for improvement, especially long term improvement, of glycemic control in patients with type 2 diabetes mellitus, especially in patients with late stage type 2 diabetes mellitus, in particular in patients additionally diagnosed of overweight, obesity (including class I, class II and/or class III obesity), visceral obesity and/or abdominal obesity being treated with a neuroleptic agent, such as a typical or atypical neuroleptic agent.

In the following preferred ranges of the amount of the SGLT2 inhibitor and the neuroleptic to be employed in the pharmaceutical composition and the methods and uses according to this invention are described. These ranges

refer to the amounts to be administered per day with respect to an adult patient, in particular to a human being, for example of approximately 70 kg body weight, and can be adapted accordingly with regard to an administration 1 or 2 times daily and with regard to other routes of administration and with regard to the age of the patient. The ranges of the dosage and amounts are calculated for the individual active moiety.

The preferred dosage range of the SGLT2 inhibitor is in the range from 0.5 mg to 500 mg, for example from 0.5 mg to 200 mg, for example from 1 to 100 mg, for example from 1 to 50 mg per day. The oral administration is preferred. Therefore, a dosage form for the SGLT-2 inhibitor may comprise the hereinbefore mentioned amounts, in particular from 1 to 50 mg or 1 to 25 mg. Particular dosage strengths (e.g. per tablet or capsule) are for example 1, 2.5, 5, 7.5, 10, 12.5, 15, 20, 25 or 50 mg of the compound of the formula (I), in particular of the compound (1.9). The application of the active ingredient may occur one, two or three times a day, preferably once a day.

Typical dosages for empagliflozin are 10mg and 25mg once daily. Typical dosages for dapagliflozin are 1 mg, 2.5 mg, 5 mg and 10 mg once daily, and 2.5 mg and 5 mg twice daily. Typical dosages for canagliflozin are 100 mg and 300 mg once daily, or 50 mg or 150 mg twice daily.

A minimum dosage level for the neuroleptic agent will vary depending upon the choice of agent, but is typically about 0.5 mg per day for the most potent compounds or about 20 mg per day for less potent compounds. A maximum dosage level for the neuroleptic agent is typically 30 mg per day for the most potent compounds or 200 mg per day for less potent compounds. The compounds are administered one to three times daily, preferably once or twice a day, and especially once a day.

Examples of routes of administration, form and dosage ranges for exemplary neuropeltic agents are disclosed below.
Clozapine is typically administered orally in the form of tablets and in a dosage range of 12.5-900 mg/day or 300-900 mg/day, in particular 350-420 mg/day.
Olanzapine is typically administered orally in the form of tablets and in a dosage range of 5-25 mg/day, 10-25 mg/day or 5-20 mg/day. Typical dosages for olanzapine are 2.5 mg, 5 mg, 7.5 mg, 10 mg, 15 mg and 20 mg once daily.
Ziprasidone is typically administered orally in the form of capsules and in a dosage range of 20-80 mg/twice a day or 80-160 mg/day.
Risperidone is typically administered orally in the form of solution or tablets and in a dosage range of 2-16 mg/day, in particular 2-4 mg/day or 4-12 mg/day or intra-venously in long-acting injectable form. Quetiapine fumarate is typically administered orally in the form of tablets and in a dosage range of oral tablets 50-900 mg/day or 300-900 mg/day.
Sertindole is typically administered in a dosage range of 4-24 mg/day.
Haloperidol is typically administered orally in the form of tablets and in a dosage range of 1-100 mg/day or 1-15 mg/day, in particular 5-15 mg/day.
Haloperidol Decanoate is typically administered orally by parenteral injection.
Chlorpromazine is typically administered by rectal suppositories or orally by capsules, solution or tablets, or by parenteral injection in the range of 30-800 mg/day or 200-500 mg/day.
Fluphenazine is typically administered in a dosage range of 0.5-40 mg/day or 1-5 mg/day. Fluphenazine Decanoate is typically administered by parenteral injection.
Thiothixene is typically administered orally in the form of capsules and in a dosage range of 6-60 mg/day or 8-30 mg/day.
Thiothixene hydrochloride is typically administered orally or parentally in the form of a solution or injection, respectively.
Trifluoperazine is typically administered in a dosage range of 2-40 mg/day.
Perphenazine is typically administered orally in the form of solution or tablets and in a dosage range of 12-64 mg/day or 16-64 mg/day.
Thioridazine is typically administered orally in the form of suspension, solution or tablets and in a dosage range of 150-800 mg/day or 100-300 mg/day.
Mesoridazine is typically administered in a dosage range of 30-400 mg/day.
Molindone is typically administered in a dosage range of 50-225 mg/day or 15-150 mg/day.
Molindone hydrochloride is typically administered orally in the form of solution
Loxapine is typically administered in a dosage range of 20-250 mg/day or 60-100 mg/dav.
Loxapine hydrochloride is typically administered orally or parentally in the form of solution or injection.
Loxapine succinate is typically administered orally in the form of capsules.
Pimozide is typically administered in a dosage range of 1-10 mg/day.

In the uses according to the present invention the neuroleptic agent and the SGLT2 inhibitor are administered in combination or alternation or sequentially. The term "administration in combination" means that the active ingredients are administered at the same time, i.e. simultaneously, or essentially at the same time. The term "administration in alternation" means that at first one of the two active ingredients, i.e. the SGLT2 inhibitor or the neuroleptic agent, is administered and after a period of time the other active ingredient, i.e. the neuroleptic agent or the SGLT2 inhibitor, is administered whereby this administration scheme may be repeated one or more times. The period of time between the administration of the first and of the second active ingredient may be in the range from 1 min to 12 hours. The administration

which is in combination or in alternation may be once, twice, three times or four times daily, preferably once or twice daily. The term "sequentially" means that to a patient the first active ingredient, in particular the neuroleptic agent, is administered to the patient one or more times in a first period of time followed by an administration of the second active ingredient, in particular the SGLT2 inhibitor which is administered to the patient one or more times in a second period of time. In other words, the term "sequentially" includes a first therapy, in particular with the neuroleptic agent, in a first period of time followed by a second therapy, in particular with the SGLT2 inhibitor, in a second period of time.

A pharmaceutical composition which is present as a separate or multiple dosage form, preferably as a kit of parts, is useful in combination therapy to flexibly suit the individual therapeutic needs of the patient.

A pharmaceutical composition may be formulated for oral, parenteral (including subcutaneous) or other routes of administration in liquid or solid form. Oral administration of the SGLT2 inhibitor is preferred. The formulations may, where appropriate, be conveniently presented in discrete dosage units and may be prepared by any of the methods well known in the art of pharmacy. All methods include the step of bringing into association the active ingredient with one or more pharmaceutically acceptable carriers, like liquid carriers or finely divided solid carriers or both, and then, if necessary, shaping the product into the desired formulation. Examples of pharmaceutical compositions comprising the SGLT2 inhibitor compound (1.9) are described in WO 2010/092126.

The pharmaceutical composition may be formulated in the form of solutions, suspensions, emulsions, tablets, granules, fine granules, powders, capsules, caplets, soft capsules, pills, oral solutions, syrups, dry syrups, chewable tablets, troches, effervescent tablets, drops, fast dissolving tablets, oral fast-dispersing tablets. Preferably the pharmaceutical composition of the SGLT2 inhibitor is in the form of tablets.

A pharmaceutical composition and dosage forms preferably comprises one or more pharmaceutical acceptable carriers. Preferred carriers must be "acceptable" in the sense of being compatible with the other ingredients of the formulation and not deleterious to the recipient thereof. Examples of pharmaceutically acceptable carriers are known to the one skilled in the art.

A pharmaceutical composition may also be formulated for parenteral administration (e.g. by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulatory agents such as suspending, stabilizing and/or dispersing agents. Alternatively, the active ingredients may be in powder form, obtained by aseptic isolation of sterile solid or by lyophilisation from solution, for constitution with a suitable vehicle, e.g. sterile, pyrogen-free water, before use. Injectable formulations may be prepared according to known formulation techniques, e.g. using suitable liquid carriers, which usually comprise sterile water, and, optionally, further additives such as e.g. preservatives, pH adjusting agents, buffering agents, isotoning agents, solubility aids and/or tensides or the like, to obtain injectable solutions or suspensions. In addition, injectable formulations may comprise further additives, for example salts, solubility modifying agents or precipitating agents which retard release of the drug(s).

For further details on dosage forms, formulations and administration of SGLT2 inhibitors of this invention and/or neuroleptic agent of this invention, reference is made to scientific literature and/or published patent documents, particularly to those cited herein.

Pharmaceutical compositions (or formulations) may be packaged in a variety of ways. Generally, an article for distribution includes one or more containers that contain the one or more pharmaceutical compositions in an appropriate form. Tablets are typically packed in an appropriate primary package for easy handling, distribution and storage and for assurance of proper stability of the composition at prolonged contact with the environment during storage. Primary containers for tablets may be bottles or blister packs.

Solutions for injection may be available in typical suitable presentation forms such as vials, cartridges or prefilled (disposable) pens, which may be further packaged.

The article may further comprise a label or package insert, which refers to instructions customarily included in commercial packages of therapeutic products, that may contain information about the indications, usage, dosage, administration, contraindications and/or warnings concerning the use of such therapeutic products. In one embodiment, the label or package inserts indicates that the composition can be used for any of the purposes described hereinbefore or hereinafter.

Methods for the manufacture of SGLT2 inhibitors according to this invention and of prodrugs thereof are known to the one skilled in the art. Advantageously, the compounds according to this invention can be prepared using synthetic methods as described in the literature, including patent applications as cited hereinbefore. Methods of manufacture are described in the WO 2006/120208 and WO 2007/031548. With regard to the preferred compound (1.9) an advantageous crystalline form is described in the international patent application WO 2006/117359 and WO 2011/039108.

The active ingredients may be present in the form of a pharmaceutically acceptable salt. The active ingredients or a pharmaceutically acceptable salt thereof may be present in the form of a solvate such as a hydrate or alcohol adduct.

Any of the above mentioned combinations and methods within the scope of the invention may be tested using animal models known in the art.

For example, methods according to this invention can be tested in genetically hyperinsulinemic or diabetic animals like db/db mice, ob/ob mice, Zucker Fatty (fa/fa) rats or Zucker Diabetic Fatty (ZDF) rats. In addition, they can be tested in animals with experimentally induced diabetes like HanWistar or Sprague Dawley rats pretreated with streptozotocin.

The effect on glycemic control of the uses and compositions according to this invention can be tested after single dosing of the SGLT2 inhibitor and the neuroleptic agent alone and in combination in an oral glucose tolerance test in the animal models described hereinbefore. The time course of blood glucose can be followed after an oral glucose challenge in overnight fasted animals. In addition, after multiple dosing of the SGLT2 inhibitor and the neuroleptic agent alone and in combination in the animal models described hereinbefore, the effect on glycemic control can be determined by measuring the HbA1c value in blood. In such experiments body weight, blood pressure and various metabolic markers can also be determined. Accordingly, the effects of chronic administration of an SGLT-2 inhibitor and a neuroleptic agent, alone and in combination, on body weight, food and water intake, blood pressure and various metabolic markers could be evaluated in animal models.

The invention is further described in the following examples, which are not intended to limit the scope of the invention.

## Pharmacological Examples

### Example 1: Oral glucose tolerance test in ZDF rats

An oral glucose tolerance test is performed in overnight fasted 9-weeks old male Zucker Diabetic Fatty (ZDF) rats (ZDF/Crl-$Lepr^{fa}$). A pre-dose blood sample is obtained by tail bleed. Blood glucose is measured with a glucometer, and the animals are randomized for blood glucose (n = 5 / group). Subsequently, the groups receive a single oral administration of either vehicle or a neuroleptic agent in the presence or absence of a SGLT-2 inhibitor. The animals receive an oral glucose load (2 g/kg) 30 min after compound administration. Blood glucose is measured in tail blood 30 min, 60 min, 90 min, 120 min, and 180 min after the glucose challenge. Glucose excursion is quantified by calculating the reactive glucose AUC. The data are presented as mean ± SEM. The two-sided unpaired Student t-test is used for statistical comparison of the control group and the active groups.
In one glucose tolerance test experiment, the SGLT-2 inhibitor is the compound (1.9) and the neuroleptic agent is olanzapine, risperidone, quetiapine, amisulpiride, aripiprazole, haloperidol, clozapine, ziprasidone, zotepine or osanetant.

### Example 2: Acute effects of antipsychotic drugs on glucose levels during a Glucose Tolerance Test

Female rats (n= 8 per group) are treated with vehicle (controls) or low- and high doses of an atypical neuroleptic agent in the presence or absence of a SGLT-2 inhibitor after overnight fasting. Before treatment with the neuroleptic agent, fasting plasma glucose is measured in each animal (time 0). Glucose levels are then tested at 60, 180 and 360 minutes after dosing. Immediately after the last glucose testing, animals are subjected to a Glucose Tolerance Test, for instance by receiving an intraperitoneal challenge injection of 1g/ml/kg of glucose. Thereafter, glucose levels are measured every 15 minutes for 2 hours.
In one glucose tolerance test experiment, the SGLT-2 inhibitor is the compound (1.9) and the neuroleptic agent is olanzapine, risperidone, quetiapine, amisulpiride, aripiprazole, haloperidol, clozapine, ziprasidone, zotepine or osanetant.

### Example 3: Treatment of hyperglycemia or type 2 diabetes

Patients receiving treatment with a neuroleptic agent and having elevated blood glucose levels or even overt type 2 diabetes are treated by a method according to the invention. Blood glucose levels of the patients are determined, and the effect of an SGLT2 inhibitor in comparison to placebo or a different therapy is assessed. This can be observed in patients treated for long periods, e.g. 3 months to 1 year or even 1 to 6 years, according to the invention. For example, the fasting glucose and/or HbA1c value is observed.

### Example 4: Oral glucose tolerance test

The aim of this study is to evaluate the acute effects of selected neuroleptic agents (clozapine, olanzapine, haloperidol) in an oral glucose tolerance test (OGTT) alone or in combination with selected SGLT-2 inhibitors (dapagliflozin, canagliflozin, empagliflozin).

**Animals**

Female Wistar rats (weight range 250-300 g upon arrival) are obtained from Janvier (Le Genest Saint Isle-France, France) and housed in pairs or three together at a temperature of 21±4°C and 55±20% humidity. The animals are maintained on a reverse phase light-dark cycle (lights off for 8 h from 09.30-17.30 h) during which time the room is illuminated by red light. The animals are housed and have free access to a fat diet and tap water until the night before the oral glucose tolerance test (OGTT) experiment.

An oral glucose tolerance test is performed in overnight fasted animals. A pre-dose blood sample (t0 -90min) is obtained by tail bleed. Blood glucose is measured with a glucometer, and the animals are randomized for blood glucose (n = 8 / group). Subsequently, the groups receive a single oral administration of either vehicle or a neuroleptic agent in the presence or absence of an SGLT-2 inhibitor. The animals receive an oral glucose load (2 g/kg) 60 min after compound administration. Blood glucose is measured in tail blood 15 min, 30 min, 60 min, 120 min, and 180 min after the glucose challenge. Glucose excursion is quantified by calculating the reactive glucose AUC. The data are presented as mean ± SEM. The two-sided unpaired Student t-test is used for statistical comparison of the control group and the active groups.

In these experiments, the SGLT-2 inhibitors dapagliflozin, canagliflozin and empagliflozin are tested at the dose of 10mg/kg po (per oral route, 5ml/kg in Natrosol 0,5%) alone or in combination with three different neuroleptic agents injected subcutaneously (in a 5% acetic acid + 7.5% 10M NaOH solution) for olanzapine (8mg/kg sc) and clozapine (8mg/kg sc), or administered intraperitonally in a 0,9% NaCl solution for haloperidol (4mg/kg).

Clozapine, olanzapine and haloperidol impaired glucose tolerance as illustrated in Figure 1A. Glucose AUCs are significantly ($p<0{,}001$) increased *versus* control with the neuroleptic agents (Figure 1B). The numbers above each bar graph in Figure 1B represent the percentage of increase in AUC over control.

In another set of experiments, SGLT-2 inhibitors are combined with the neuroleptic agents. Figure 2A illustrates the OGTT of olanzapine in combination with the SGLT-2 inhibitors. All SGLT-2 inhibitors tested reduced significantly the AUC glucose in comparison to olanzapine alone (Figure 2B). The numbers above each bar graph in Figure 2B represent the percentage of increase in AUC over control.

OGTT with SGLT-2 inhibitors in combination with clozapine are represented in Figure 3A. The SGLT-2 inhibitors improve the AUC glucose when combined with clozapine in comparison to clozapine alone (Figure 3B). The numbers above each bar graph represent the percentage increase AUC over control.

Similar effects have been observed with haloperidol (Figure 4A). But because the worsening of the OGTT was less pronounced with haloperidol compared to olanzapine and clozapine, the improvements in glucose tolerance when combined with SGLT-2 inhibitors were less pronounced (Figure 4B).

**Examples of Formulations**

The following examples of formulations, which may be obtained analogously to methods known in the art, serve to illustrate the present invention more fully without restricting it to the contents of these examples. The term "active substance" denotes an SGLT-2 inhibitor according to this invention, for example a compound of the formula (1.9) or its crystalline form (I.9X).

The active pharmaceutical ingredient or active sustance, i.e. the compound (1.9), preferably in the crystalline form (I9.X), is milled with a suitable mill like pin- or jet-mill in order to obtain the desired particle size distribution before manufacturing of the pharmaceutical composition or dosage form.

Examples of typical particle size distribution values X90, X50 and X10 for the preferred active pharmaceutical ingredient according to the invention are shown in the table below.

Typical particle size distribution results

| | Active substance Batch 1 | Active substance Batch 2 |
|---|---|---|
| X10 | 1,8 μm | 1,7 μm |
| X50 | 18,9 μm | 12,1 μm |
| X90 | 45,3 μm | 25,9 μm |

Example 1: Dry ampoule containing 50 mg of active substance per 10 ml

Composition:

| | |
|---|---|
| Active substance | 50.0 mg |

(continued)

| | |
|---|---|
| Mannitol | 50.0 mg |
| water for injections | ad 10.0 ml |

Preparation:
Active substance and mannitol are dissolved in water. After packaging the solution is freeze-dried. To produce the solution ready for use, the product is dissolved in water for injections.

Example 2: Dry ampoule containing 25 mg of active substance per 2 ml

Composition:

| | |
|---|---|
| Active substance | 25.0 mg |
| Mannitol | 100.0 mg |
| water for injections | ad 2.0 ml |

Preparation:
Active substance and mannitol are dissolved in water. After packaging, the solution is freeze-dried. To produce the solution ready for use, the product is dissolved in water for injections.

Example 3: Tablet containing 50 mg of active substance

Composition:

| | |
|---|---|
| (1) Active substance | 50.0 mg |
| (2) Mannitol | 98.0 mg |
| (3) Maize starch | 50.0 mg |
| (4) Polyvinylpyrrolidone | 15.0 mg |
| (5) Magnesium stearate | 2.0 mg |
| | 215.0 mg |

Preparation:
(1), (2) and (3) are mixed together and granulated with an aqueous solution of (4). (5) is added to the dried granulated material. From this mixture tablets are pressed, biplanar, faceted on both sides and with a dividing notch on one side. Diameter of the tablets: 9 mm.

Example 4: Capsules containing 50 mg of active substance

Composition:

| | |
|---|---|
| (1) Active substance | 50.0 mg |
| (2) Dried maize starch | 58.0 mg |
| (3) Mannitol | 50.0 mg |
| (4) Magnesium stearate | 2.0 mg |
| | 160.0 mg |

Preparation:
(1) is triturated with (3). This trituration is added to the mixture of (2) and (4) with vigorous mixing. This powder mixture is packed into size 3 hard gelatin capsules in a capsule filling machine.

Example 5: Tablets containing 2.5mg, 5mg, 10mg, 25mg, 50mg of active substance

| **Active substance** | 2.5 mg Mg/per tablet | 5 mg Mg/per tablet | 10 mg Mg/per tablet | 25 mg Mg/per tablet | 50 mg Mg/per tablet |
|---|---|---|---|---|---|
| **Wet granulation** | | | | | |
| active substance | 2.5000 | 5.000 | 10.00 | 25.00 | 50.00 |
| Lactose Monohydrate | 40.6250 | 81.250 | 162.50 | 113.00 | 226.00 |
| Microcrystalline Cellulose | 12.5000 | 25.000 | 50.00 | 40.00 | 80.00 |
| Hydroxypropyl Cellulose | 1.8750 | 3.750 | 7.50 | 6.00 | 12.00 |
| Croscarmellose Sodium | 1.2500 | 2.500 | 5.00 | 4.00 | 8.00 |
| Purified Water | q.s. | q.s. | q.s. | q.s. | q.s. |
| **Dry Adds** | | | | | |
| Microcrystalline Cellulose | 3.1250 | 6.250 | 12.50 | 10.00 | 20.00 |
| Colloidal silicon dioxide | 0.3125 | 0.625 | 1.25 | 1.00 | 2.00 |
| Magnesium stearate | 0.3125 | 0.625 | 1.25 | 1.00 | 2.00 |
| Total core | 62.5000 | 125.000 | 250.00 | 200.00 | 400.00 |
| **Film Coating** | | | | | |
| Film coating system | 2.5000 | 4.000 | 7.00 | 6.00 | 9.00 |
| Purified Water | q.s. | q.s. | q.s. | q.s. | q.s. |
| **Total** | **65.000** | **129.000** | **257.00** | **206.00** | **409.00** |

Example 6: Manufacturing process for tablets

| **Step** | **EQUIPMENT** | **MATERIALS** | | **OPERATION** | **IN-PROCESS CONTROLS** |
|---|---|---|---|---|---|
| 1 | Screen, blender and high shear granulator | Active substance | → | MIX | |
| | | Hydroxypropyl Cellulose (screened) | → | | |
| | | Croscarmellose Sodium | → | | |
| | | Part of Microcrystalline Cellulose (PH102) | → | | |
| | | Lactose Monohydrate | → | | |

(continued)

| Step | EQUIPMENT | MATERIALS | | OPERATION | IN-PROCESS CONTROLS |
|---|---|---|---|---|---|
| 2 | High shear granulator | Purified Water | → | GRANULATE | |
| | | | | ↓ | |
| 3 | Fluid bed drier | | | DISCHARGE ONTO DRYER AND DRY | LOD ≤ 2.0% at 100°C |
| | | | | ↓ | |
| 4 | Mill | | | DRY MILL | |
| | | | | ↓ | |
| 5 | Mill, blender | Colloidal Silicon Dioxide + Microcrystalline Cellulose (PH102) | → | MIX | |
| | | | | ↓ | |
| 6 | Mill, blender | Magnesium Stearate | → | MIX | |
| | | | | ↓ | |
| | | | | Final tablet blend | |
| | | | | ↓ | |
| 7 | Tablet press | | | COMPRESS INTO TABLETS | Tablet weight, height, crushing strength, friability, disintegration |
| | | | | ↓ | |
| | | | | Core tablets | |
| | | | | ↓ | |
| 8 | Propeller Stirrer Drum coater | Suspend film-coating system in water and mix | → | FILM COATING | |
| | | | | ↓ | |
| | | | | Final film coated tablets | Tablet weight, height, crushing strength, disintegration |

Example 7: Pharmaceutical composition containing other fillers

Copovidone is dissolved in purified water at ambient temperature to produce a granulation liquid. A glucopyranosyl-substituted benzene derivative according to the present invention, mannitol, pregelatinized starch and corn starch are blended in a suitable mixer, to produce a pre-mix. The pre-mix is moistened with the granulation liquid and subsequently granulated. The moist granulate is sieved through a suitable sieve. The granulate is dried at about 60 °C inlet air temperature in a fluid bed dryer until a loss on drying value of 1-4 % is obtained. The dried granulate is sieved through a sieve with a mesh size of 1.0 mm.

Magnesium stearate is passed through a sieve for delumping and added to the granulate. Subsequently the final blend is produced by final blending in a suitable blender for three minutes and compressed into tablet cores.

Hydroxypropyl methylcellulose, polyethylene glycol, talc, titanium dioxide and iron oxide are suspended in purified water in a suitable mixer at ambient temperature to produce a coating suspension. The tablet cores are coated with the coating suspension to a weight gain of about 3 % to produce film-coated tablets. The following formulation variants can be obtained:

| Ingredient | mg / tablet | mg / tablet | mg / tablet | mg / tablet | mg / tablet |
|---|---|---|---|---|---|
| Active substance | 2.5 | 5.0 | 10.0 | 25.0 | 50.0 |
| Mannitol | 133.4 | 130.9 | 125.9 | 110.9 | 221.8 |
| Pregelatinised starch | 18.0 | 18.0 | 18.0 | 18.0 | 36.0 |
| Maize starch | 18.0 | 18.0 | 18.0 | 18.0 | 36.0 |
| Copovidone | 5.4 | 5.4 | 5.4 | 5.4 | 10.8 |
| Magnesium stearate | 2.7 | 2.7 | 2.7 | 2.7 | 5.4 |
| Film coat | 5.0 | 5.0 | 5.0 | 5.0 | 10.0 |
| **Total** | **185.0** | **185.0** | **185.0** | **185.0** | **370.0** |

Example 8: Pharmaceutical composition containg other disintegrant

Copovidone is dissolved in purified water at ambient temperature to produce a granulation liquid. An glucopyranosyl-substituted benzene derivative according to the present invention, mannitol, pregelatinized starch and corn starch are blended in a suitable mixer, to produce a pre-mix. The pre-mix is moistened with the granulation liquid and subsequently granulated. The moist granulate is sieved through a suitable sieve. The granulate is dried at about 60 °C inlet air temperature in a fluid bed dryer until a loss on drying value of 1-4 % is obtained. The dried granulate is sieved through a sieve with a mesh size of 1.0 mm.

Crospovidone is added to the dried granulate and mixed for 5 minutes to produce the main blend. Magnesium stearate is passed through a sieve for delumping and added to main blend. Subsequently the final blend is produced by final blending in a suitable blender for three minutes and compressed into 8 mm round tablet cores with a compression force of 16 kN.

Hydroxypropyl methylcellulose, polyethylene glycol, talc, titanium dioxide and iron oxide are suspended in purified water in a suitable mixer at ambient temperature to produce a coating suspension. The tablet cores are coated with the coating suspension to a weight gain of about 3 % to produce film-coated tablets. The following formulation variants can be obtained:

| Ingredient | mg / tablet | mg / tablet | mg / tablet | mg / tablet | mg / tablet |
|---|---|---|---|---|---|
| Active substance | 2.5 | 5.0 | 10.0 | 25.0 | 50.0 |
| Mannitol | 127.5 | 125.0 | 120.0 | 105.0 | 210.0 |
| Microcrystalline Cellulose | 39.0 | 39.0 | 39.0 | 39.0 | 78.0 |
| Crospovidone | 2.0 | 2.0 | 2.0 | 2.0 | 4.0 |
| Copovidone | 5.4 | 5.4 | 5.4 | 5.4 | 10.8 |
| Magnesium stearate | 3.6 | 3.6 | 3.6 | 3.6 | 7.2 |
| Film coat | 5.0 | 5.0 | 5.0 | 5.0 | 10.0 |
| **Total** | **185.0** | **185.0** | **185.0** | **185.0** | **370.0** |

The tablet hardness, the friability, the content uniformity, the disintegration time and the dissolution properties are determined as described hereinbefore.

Example 9: Direct compression formulation

1. Screen the active ingredient, microcrystalline cellulose, croscarmellose.sodium and either hydroxypropyl cellulose or polyethylene glycol powder through a 20 mesh hand screen.
2. Add the above items into the high shear mixer and mix for two minutes.
3. Make a premix (~1/1) of the lactose and colloidal silicon dioxide.

4. Screen the premix through a 20 mesh hand screen and add to the mixer.
5. Screen the remaining lactose through a 20 mesh hand screen and add to the mixer.
6. Mix in components in the mixer for 2 minutes.
7. Screen the magnesium stearate through a 30 mesh hand screen and add to the mixer.
8. Mix for 1 minute 30 seconds to obtain the final blend.
9 Tabletting of the final blend on a suitable tabletting press.
10. Optionally film coating of the tablet cores.

| Ingredient | mg / tablet | mg / tablet | mg / tablet | mg / tablet | mg / tablet |
|---|---|---|---|---|---|
| Active substance | 2.5000 | 5.000 | 10.00 | 25.0 | 50.0 |
| Lactose Monohydrate | 43.7500 | 87.500 | 175.00 | 74.0 | 148.0 |
| Microcrystalline Cellulose | 12.5000 | 25.000 | 50.00 | 80.0 | 160.0 |
| Polyethylene glycol | - | - | - | 10.0 | 20.0 |
| Croscarmellose sodium | 1.2500 | 2.500 | 5.00 | 8.0 | 16.0 |
| Hydroxypropyl cellulose | 1.8750 | 3.750 | 7.50 | - | - |
| Colloidal Silicon dioxide | 0.3125 | 0.625 | 1.25 | 1.0 | 2.0 |
| Magnesium stearate | 0.3125 | 0.625 | 1.25 | 2.0 | 4.0 |
| Film coat | 2.5000 | 4.000 | 7.00 | 6.00 | 9.00 |
| Purified water | q.s. | q.s. | q.s. | q.s. | q.s. |
| **Total** | **65.000** | **129.000** | **257.00** | **206.00** | **409.00** |

Example 10: Tablets containing 0.5mg, 5mg, 25mg, 100mg of active substance

| **Active substance** | 0.5 mg mg/per tablet | 5 mg mg/per tablet | 25 mg mg/per tablet | 100 mg mg/per tablet |
|---|---|---|---|---|
| **Wet granulation** | | | | |
| active substance | 2.5000 | 5.000 | 25.00 | 100.00 |
| Lactose Monohydrate | 60.00 | 55.00 | 42.00 | 168.00 |
| Microcrystalline Cellulose | 20.00 | 20.00 | 38.00 | 152.00 |
| Hyd roxypropyl Cellulose | 5.00 | 5.00 | 7.50 | 30.00 |
| Croscarmellose Sodium | 4.00 | 4.00 | 6.00 | 24.00 |
| Purified Water | q.s. | q.s. | q.s. | q.s. |
| **Dry Adds** | | | | |
| Microcrystalline Cellulose | 10.00 | 10.00 | 30.00 | 120.00 |
| Colloidal silicon dioxide | -- | 0.50 | 0.75 | 3.00 |
| Magnesium stearate | 0.50 | 0.50 | 0.75 | 3.00 |
| **Total** | **100.00** | **100.00** | **150.00** | **600.00** |

The active substance, e.g. the compound (I.9), preferably in the crystalline form (I.9X), hydroxypropyl cellulose, and croscarmellose sodium are mixed in a blender. This premix is mixed with lactose monohydrate and a portion of microcrystalline cellulose. The resulting blend is granulated with purified water. Multiple granulation subparts may be

produced for an individual tablet batch, as needed, depending on the batch size and equipment used.
The granulation is discharged onto dryer trays and dried. The granulation is then milled. The remainder of the microcrystalline cellulose is added (as a premix with the colloidal silicon dioxide for all strengths other than the 0.5 mg) to the milled granulation, and mixed. The magnesium stearate is premixed with a portion of the blend, screened into the remainder of the granulation, and mixed.
The final tablet blend is compressed into tablets using a tablet press. The finished tablets are packaged using a suitable container closure system.

Example 11: Tablets containing 1mg, 5mg, 25mg of active substance

| **Active substance** | 1 mg mg/per tablet | 5 mg mg/per tablet | 25 mg mg/per tablet |
|---|---|---|---|
| **Wet granulation** | | | |
| active substance | 1.00 | 5.00 | 25.00 |
| Lactose Monohydrate | 63.00 | 59.00 | 39.00 |
| Microcrystalline Cellulose | 20.00 | 20.00 | 20.00 |
| Hyd roxypropyl Cellulose | 3.00 | 3.00 | 3.00 |
| Croscarmellose Sodium | 2.00 | 2.00 | 2.00 |
| Purified Water | q.s. | q.s. | q.s. |
| **Dry Adds** | | | |
| Microcrystalline Cellulose | 10.00 | 10.00 | 10.00 |
| Colloidal silicon dioxide | 0.50 | 0.50 | 0.50 |
| Magnesium stearate | 0.50 | 0.50 | 0.50 |
| **Total** | **100.00** | **100.00** | **100.00** |

The active substance, e.g. the compound (1.9), preferably in the crystalline form (I.9X), is passed through a screen and added to a blender or a high shear granulator. The hydroxypropyl cellulose and croscarmellose sodium are passed through a screen, added to the drug substance, and mixed. The intra-granular portion of microcrystalline cellulose is passed through a screen into a high shear granulator and mixed with the drug substance premix. Lactose is then added by passing the material through a screen into the granulator and mixing. The resulting blend is granulated with purified water. For larger batches, multiple granulation subparts may be produced for an individual tablet batch, as needed, depending on the batch size and equipment used.
The granulation is discharged onto dryer trays and dried. The granulation is then passed through a mill into a blender. The colloidal silicon dioxide is pre-mixed with a portion of the extra-granular microcrystalline cellulose. This premix is passed through a mill into the blender, followed by the remaining extra-granular microcrystalline cellulose, and mixed with the milled granulation. The magnesium stearate is premixed with a portion of the blend, passed through a mill into the remainder of the granulation, and mixed.
The final tablet blend is compressed into tablets using a tablet press. The finished tablets are packaged using a suitable container closure system.

## Claims

1. A SGLT2 inhibitor for use in a method for improving glycemic control in a patient treated for a psychotic disorder with a neuroleptic agent, **characterized in that** the SGLT2 inhibitor is selected from the group consisting of dapagliflozin, canagliflozin and empagliflozin and **in that** the neuroleptic agent and the SGLT2 inhibitor are administered to the patient.

2. The SGLT2 inhibitor for the use according to claim 1 wherein the patient is diagnosed with impaired glucose tolerance (IGT), impaired fasting blood glucose (IFG) with insulin resistance, with metabolic syndrome and/or with type 1

diabetes mellitus or type 2 diabetes mellitus.

3. The SGLT2 inhibitor for the use according to claim 1 or 2, wherein said patient:

(1) is an individual diagnosed of one or more of the conditions selected from the group consisting of overweight, obesity, visceral obesity and abdominal obesity; or
(2) is an individual who shows one, two or more of the following conditions:

(a) a fasting blood glucose or serum glucose concentration greater than 100 mg/dL, in particular greater than 125 mg/dL;
(b) a postprandial plasma glucose equal to or greater than 140 mg/dL;
(c) an HbA1c value equal to or greater than 6.5 %, in particular equal to or greater than 8.0 %; or

(3) is an individual wherein one, two, three or more of the following conditions are present:

(a) obesity, visceral obesity and/or abdominal obesity,
(b) triglyceride blood level ≥ 150 mg/dL,
(c) HDL-cholesterol blood level < 40 mg/dL in female patients and < 50 mg/dL in male patients,
(d) a systolic blood pressure ≥ 130 mm Hg and a diastolic blood pressure ≥ 85 mm Hg,
(e) a fasting blood glucose level ≥ 100 mg/dL.

4. The SGLT2 inhibitor for the use according to claim 3, wherein said patient is an individual diagnosed of one or more of the conditions selected from the group consisting of overweight, obesity, visceral obesity and abdominal obesity.

5. The SGLT2 inhibitor for the use according to one or more of the claim 1 to 4, wherein the neuroleptic agent is a Phenothiazine, a Thioxanthene, a Butyrophenone, a Dibenzoxazepine, a Dihydroindolone, a Diphenylbutylpiperidine, or a Benzisoxazole.

6. The SGLT2 inhibitor for the use according to one or more of the claim 1 to 4, wherein the neuroleptic agent is olanzapine, risperidone, quetiapine, amisulpiride, aripiprazole, haloperidol, clozapine, ziprasidone, zotepine, paliperidone or osanetant.

7. The SGLT2 inhibitor for the use according to one or more of the claim 1 to 6, wherein said SGLT-2 inhibitor and said neuroleptic agent are administered in combination or alternation or sequentially to the patient.

8. The SGLT2 inhibitor for the use according to one or more of the claim 1 to 7, wherein said SGLT-2 inhibitor is empagliflozin.

9. A pharmaceutical composition comprising (a) a neuroleptic agent and (b) an SGLT2 inhibitor selected from the group consisting of dapagliflozin, canagliflozin and empagliflozin.

10. The pharmaceutical composition according to claim 9, wherein the neuroleptic agent is a Phenothiazine, a Thioxanthene, a Butyrophenone, a Dibenzoxazepine, a Dihydroindolone, a Diphenylbutylpiperidine, or a Benzisoxazole.

11. The pharmaceutical composition according to claim 9, wherein the neuroleptic agent is olanzapine, risperidone, quetiapine, amisulpiride, aripiprazole, haloperidol, clozapine, ziprasidone, zotepine, paliperidone or osanetant.

12. The pharmaceutical composition according to one or more of the claims 9, 10 and 11, wherein the SGLT2 inhibitor is empagliflozin.

## Patentansprüche

1. SGLT2-Inhibitor zur Verwendung in einem Verfahren zur Verbesserung der glykämischen Kontrolle bei einem Patienten, der mit einem neuroleptischen Mittel gegen eine psychotische Störung behandelt wird, **dadurch gekennzeichnet, dass** der SGLT2-Inhibitor ausgewählt ist aus der Gruppe, bestehend aus Dapagliflozin, Canagliflozin und Empagliflozin, und dadurch, dass das neuroleptische Mittel und der SGLT2-Inhibitor an den Patienten verabreicht werden.

2. SGLT2-Inhibitor zur Verwendung nach Anspruch 1, wobei bei dem Patienten gestörte Glukosetoleranz (IGT), gestörte Nüchternblutglucose bzw. gestörter Nüchternblutzucker (IFG) mit Insulinresistenz, metabolisches Syndrom und/oder Diabetes mellitus Typ 1 oder Typ 2 diagnostiziert wurde.

3. SGLT2-Inhibitor zur Verwendung nach Anspruch 1 oder 2, wobei der Patient:

(1) ein Individuum darstellt, bei dem ein oder mehrere der Zustände, ausgewählt aus der Gruppe, bestehend aus Übergewicht, Adipositas, viszerale Adipositas und abdominale Adipositas, diagnostiziert wurde; oder
(2) ein Individuum darstellt, das ein, zwei oder mehr der nachfolgenden Bedingungen zeigt:

(a) eine Nüchternblutglucose- bzw. Nüchternblutzuckerkonzentration oder Serumblutzucker- bzw. Serumglucosekonzentration höher als 100 mg/dL, insbesondere höher als 125 mg/dL;
(b) eine postprandiale Plasmaglucose bzw. ein postprandialer Plasmazucker größer oder gleich 140 mg/dL;
(c) einen HbA1c-Wert größer oder gleich 6,5%, insbesondere größer oder gleich 8,0%; oder

(3) ein Individuum darstellt, wobei ein, zwei, drei oder mehr der nachfolgenden Bedingungen vorliegen:

(a) Adipositas, viszerale Adipositas und/oder abdominale Adipositas,
(b) Triglyceridblutspiegel ≥ 150 mg/dL,
(c) HDL-Cholesterinblutspiegel < 40 mg/dL bei weiblichen Patienten und < 50 mg/dL bei männlichen Patienten,
(d) ein systolischer Blutdruck ≥ 130 mm Hg und einen diastolischen Blutdruck ≥ 85 mm Hg,
(e) ein Nüchternblutzucker- bzw. Nüchternblutglucosespiegel ≥ 100 mg/dL.

4. SGLT2-Inhibitor zur Verwendung nach Anspruch 3, wobei der Patient ein Individuum darstellt, bei dem ein oder mehrere der Zustände, ausgewählt aus der Gruppe, bestehend aus Übergewicht, Adipositas, viszerale Adipositas und abdominale Adipositas, diagnostiziert wurden.

5. SGLT2-Inhibitor zur Verwendung nach einem oder mehreren der Ansprüche 1 bis 4, wobei das neuroleptische Mittel ein Phenothiazin, ein Thioxanthen, ein Butyrophenon, ein Dibenzoxazepin, ein Dihydroindolon, ein Diphenylbutylpiperidin oder ein Benzisoxazol darstellt.

6. SGLT2-Inhibitor zur Verwendung nach einem oder mehreren der Ansprüche 1 bis 4, wobei das neuroleptische Mittel Olanzapin, Risperidon, Quetiapin, Amisulpirid, Aripiprazol, Haloperidol, Clozapin, Ziprasidon, Zotepin, Paliperidon oder Osanetant darstellt.

7. SGLT2-Inhibitor zur Verwendung nach einem oder mehreren der Ansprüche 1 bis 6, wobei der SGLT-2-Inhibitor und das neuroleptische Mittel in Kombination oder abwechselnd oder aufeinanderfolgend an den Patienten verabreicht werden.

8. SGLT2-Inhibitor zur Verwendung nach einem oder mehreren der Ansprüche 1 bis 7, wobei der SGLT-2-Inhibitor Empagliflozin darstellt.

9. Pharmazeutische Zusammensetzung, umfassend (a) ein neuroleptisches Mittel und (b) einen SGLT2-Inhibitor, ausgewählt aus der Gruppe, bestehend aus Dapagliflozin, Canagliflozin und Empagliflozin.

10. Pharmazeutische Zusammensetzung, nach Anspruch 9, wobei das neuroleptische Mittel ein Phenothiazin, ein Thioxanthen, ein Butyrophenon, ein Dibenzoxazepin, ein Dihydroindolon, ein Diphenylbutylpiperidin oder ein Benzisoxazol darstellt.

11. Pharmazeutische Zusammensetzung, nach Anspruch 9, wobei das neuroleptische Mittel Olanzapin, Risperidon, Quetiapin, Amisulpirid, Aripiprazol, Haloperidol, Clozapin, Ziprasidon, Zotepin, Paliperidon oder Osanetant darstellt.

12. Pharmazeutische Zusammensetzung nach einem oder mehreren der Ansprüche 9, 10 und 11, wobei der SGLT2-Inhibitor Empagliflozin darstellt.

## Revendications

1. Inhibiteur du SGLT2 pour son utilisation dans une méthode d'amélioration du contrôle de la glycémie chez un patient traité pour un trouble psychotique avec un agent neuroleptique, **caractérisé en ce que** l'inhibiteur du SGLT2 est sélectionné dans le groupe consistant en la dapagliflozine, la canagliflozine et l'empagliflozine et **en ce que** l'agent neuroleptique et l'inhibiteur du SGLT2 sont administrés au patient.

2. Inhibiteur du SGLT2 pour son utilisation selon la revendication 1 dans lequel le patient est diagnostiqué avec une altération de la tolérance au glucose (ATG), une altération de la glycémie à jeun (AGJ) avec une résistance à l'insuline, avec un syndrome métabolique et/ou avec un diabète de type 1 ou un diabète de type 2.

3. Inhibiteur du SGLT2 pour son utilisation selon la revendication 1 ou 2, dans lequel ledit patient :

   (1) est un individu diagnostiqué avec une ou plusieurs des affections sélectionnées dans le groupe consistant en le surpoids, l'obésité, l'obésité viscérale et l'obésité abdominale ; ou
   (2) est un individu qui présente une, deux ou plus des affections suivantes :

      (a) une glycémie ou une concentration de glucose sérique à jeun supérieure à 100 mg/dl, en particulier supérieure à 125 mg/dl ;
      (b) un glucose plasmatique postprandial supérieur ou égal à 140 mg/dl ;
      (c) une valeur de HbA1c supérieure ou égale à 6,5 %, en particulier supérieure ou égale à 8,0 % ; ou

   (3) est un individu chez lequel une, deux, trois ou plus des affections suivantes sont présentes :

      (a) l'obésité, l'obésité viscérale et/ou l'obésité abdominale,
      (b) un taux sanguin des triglycérides ≥ 150 mg/dl,
      (c) un taux sanguin de HDL-cholestérol < 40 mg/dl chez les patientes et < 50 mg/dl chez les patients,
      (d) une pression sanguine systolique ≥ 130 mm Hg et une pression sanguine diastolique ≥ 85 mm Hg,
      (e) un taux de glycémie à jeun ≥ 100 mg/dl.

4. Inhibiteur du SGLT2 pour son utilisation selon la revendication 3, dans lequel ledit patient est un individu diagnostiqué avec une ou plusieurs des affections sélectionnées dans le groupe consistant en le surpoids, l'obésité, l'obésité viscérale et l'obésité abdominale.

5. Inhibiteur du SGLT2 pour son utilisation selon une ou plusieurs des revendications 1 à 4, dans lequel l'agent neuroleptique est une phénothiazine, un thioxanthène, une butyrophénone, une dibenzoxazépine, une dihydroindolone, une diphénylbutylpipéridine, ou un benzisoxazole.

6. Inhibiteur du SGLT2 pour son utilisation selon une ou plusieurs des revendications 1 à 4, dans lequel l'agent neuroleptique est l'olanzapine, la rispéridone, la quétiapine, l'amisulpride, l'aripiprazole, l'halopéridol, la clozapine, la ziprasidone, la zotépine, la palipéridone ou l'osanétant.

7. Inhibiteur du SGLT2 pour son utilisation selon une ou plusieurs des revendications 1 à 6, dans lequel ledit inhibiteur du SGLT2 et ledit agent neuroleptique sont administrés en combinaison ou en alternance ou séquentiellement au patient.

8. Inhibiteur du SGLT2 pour son utilisation selon une ou plusieurs des revendications 1 à 7, dans lequel ledit inhibiteur du SGLT2 est l'empagliflozine.

9. Composition pharmaceutique comprenant (a) un agent neuroleptique et (b) un inhibiteur du SGLT2 sélectionné dans le groupe consistant en la dapagliflozine, la canagliflozine et l'empagliflozine.

10. Composition pharmaceutique selon la revendication 9, dans laquelle l'agent neuroleptique est une phénothiazine, un thioxanthène, une butyrophénone, une dibenzoxazépine, une dihydroindolone, une diphénylbutylpipéridine, ou un benzisoxazole.

11. Composition pharmaceutique selon la revendication 9, dans laquelle l'agent neuroleptique est l'olanzapine, la rispéridone, la quétiapine, l'amisulpride, l'aripiprazole, l'halopéridol, la clozapine, la ziprasidone, la zotépine, la palipéridone ou l'osanétant.

12. Composition pharmaceutique selon une ou plusieurs des revendications 9, 10 et 11, dans laquelle l'inhibiteur du SGLT2 est l'empagliflozine.

## Figure 1 A
## Female SD rats (n=8)
## Oral glucose tolerance test

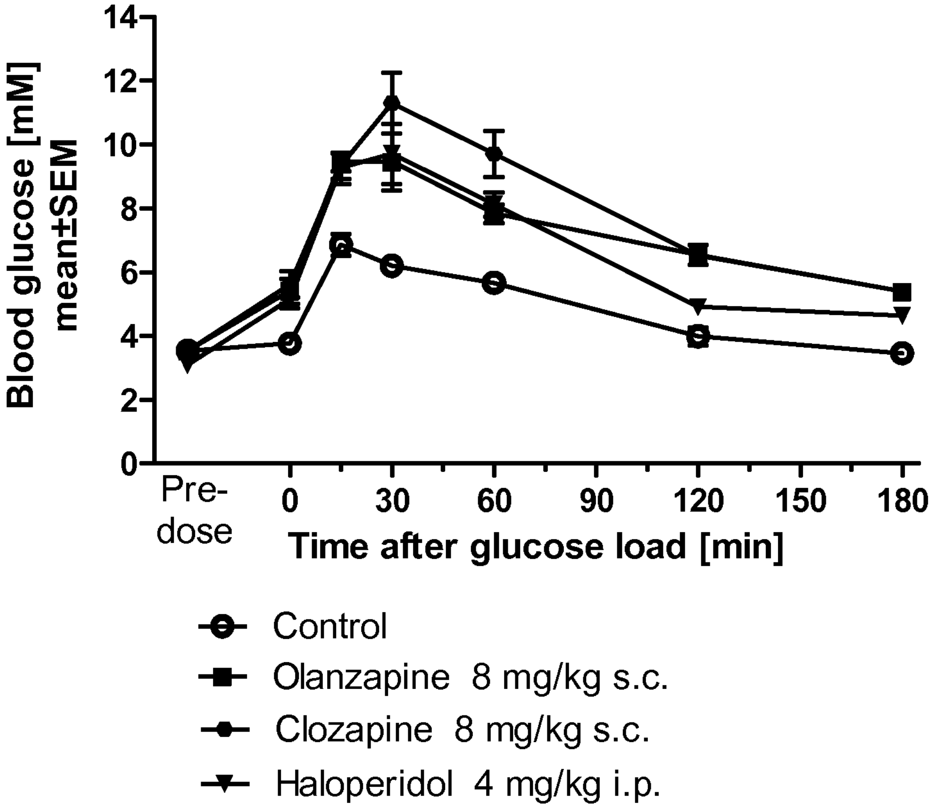

**Figure 1B**
**Female SD rats (n=8)**
**Glucose $AUC_{0\text{-}180\ min}$ with baseline (0 min)**
**(*** p<0.001 vs. Control)**

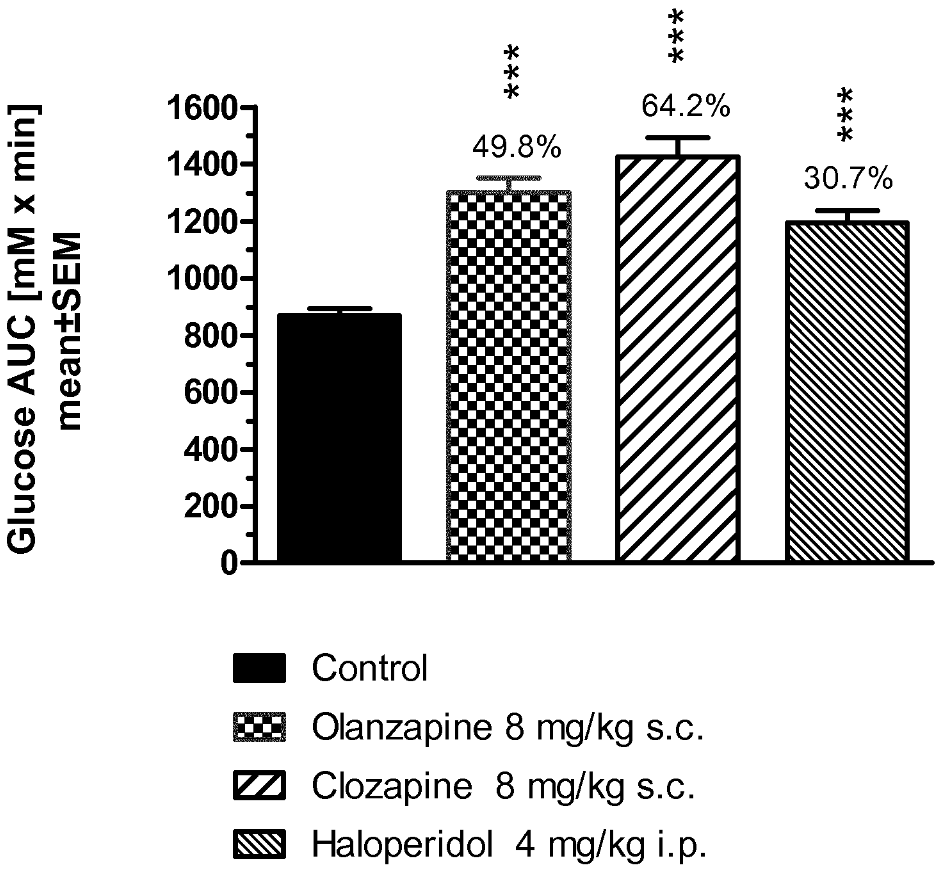

## Figure 2A
## Female SD rats (n=8)
## Oral glucose tolerance test

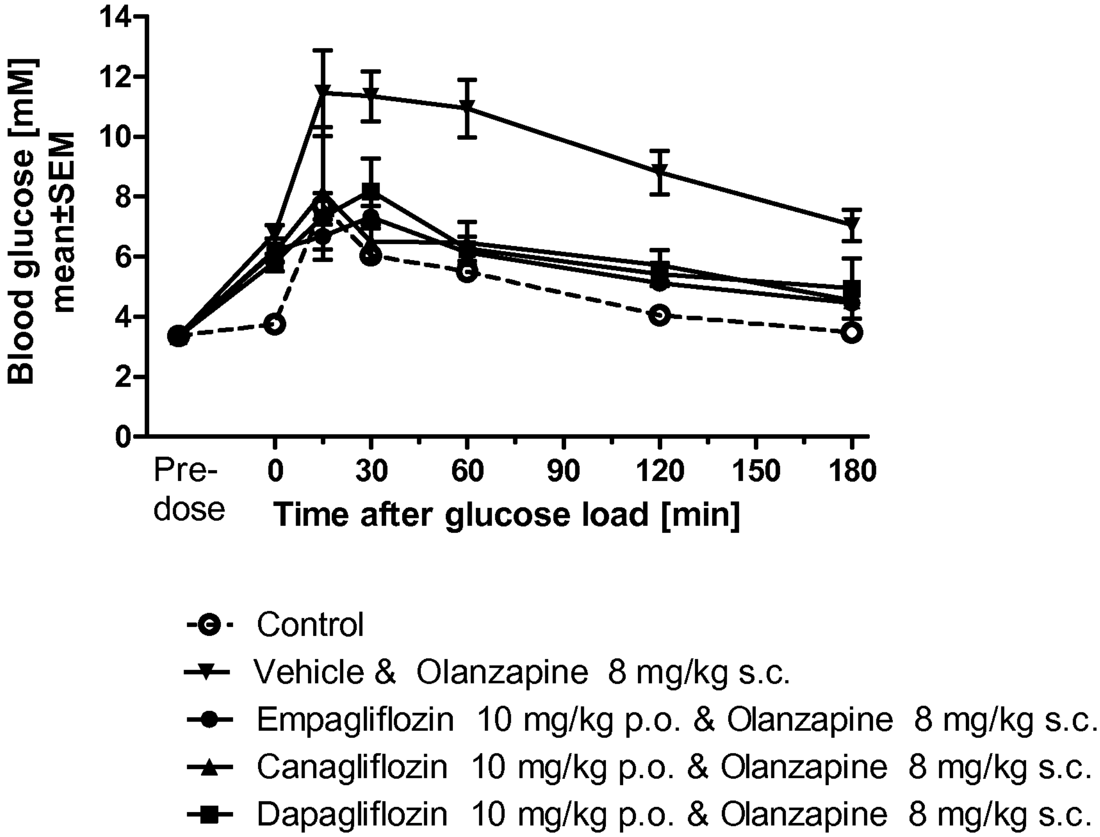

## Figure 2B

**Glucose $AUC_{0\text{-}180\ min}$ with baseline (0 min)**

**(* p<0.05;*** p<0.001 vs. Control # p<0,05; ## < 0,01; ### p<0,001 vs olanzapine)**

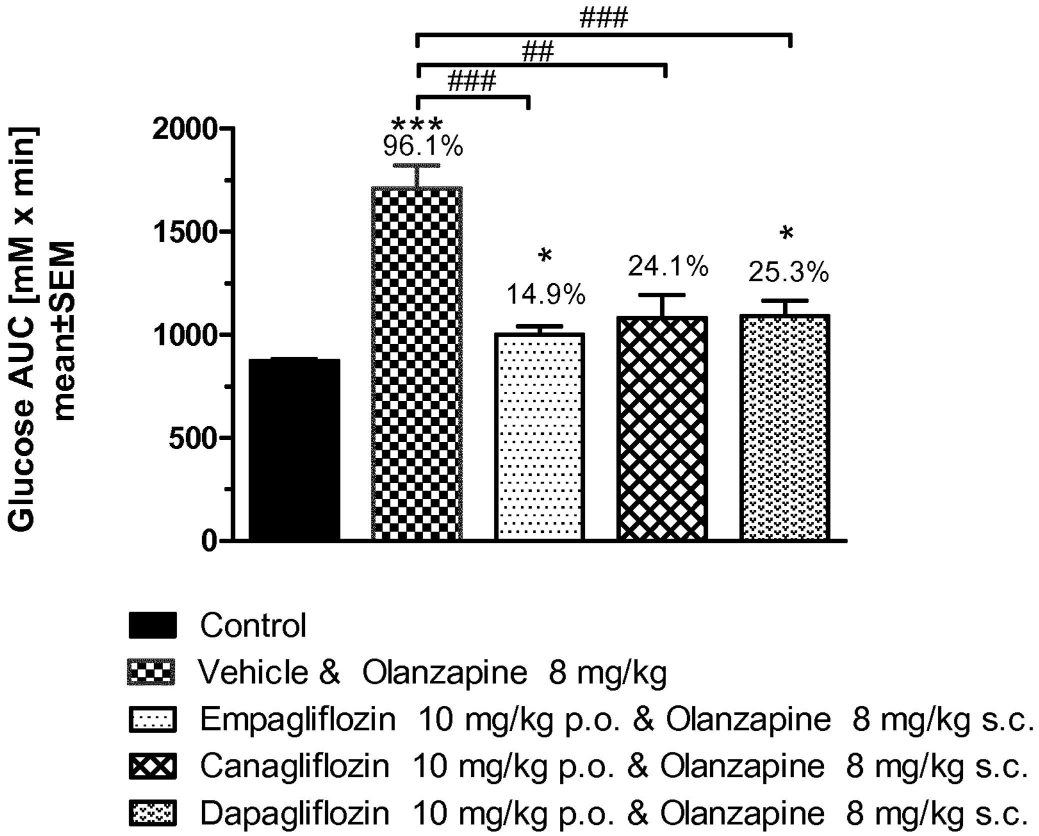

**Figure 3A**
**Female SD rats (n=8)**
**Oral glucose tolerance test**

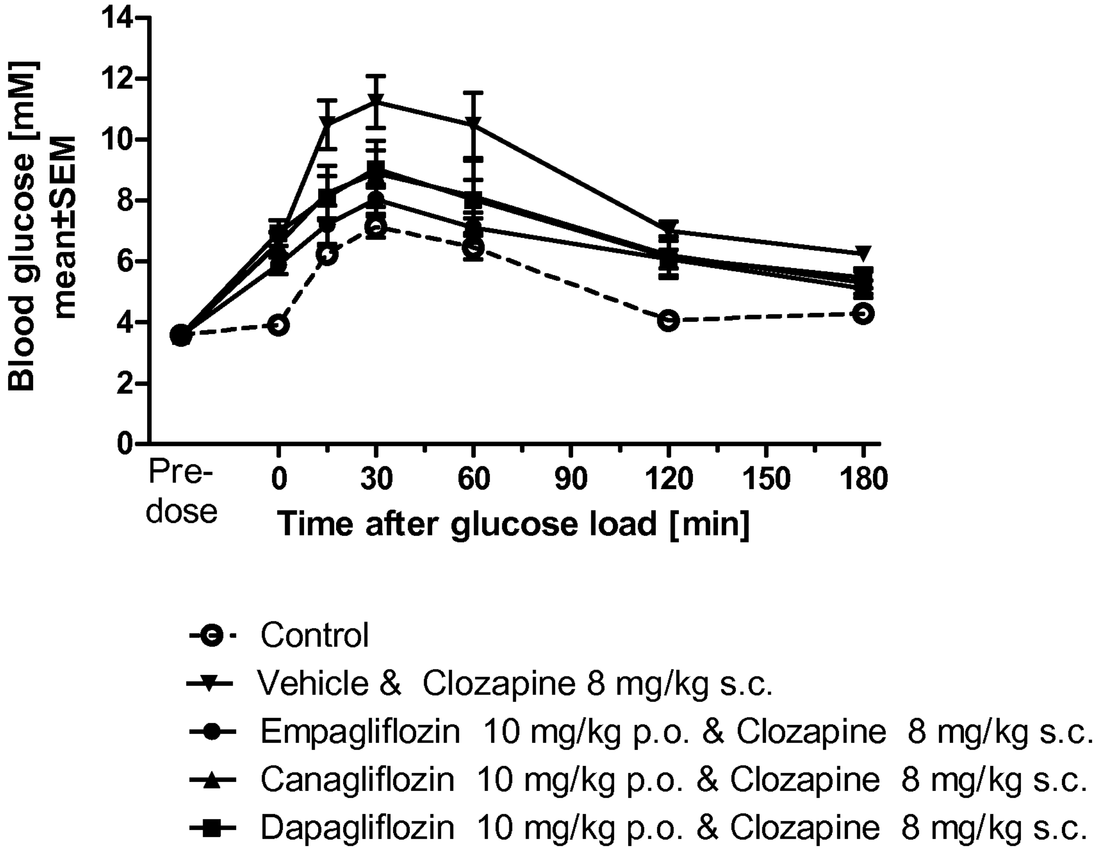

## Figure 3B

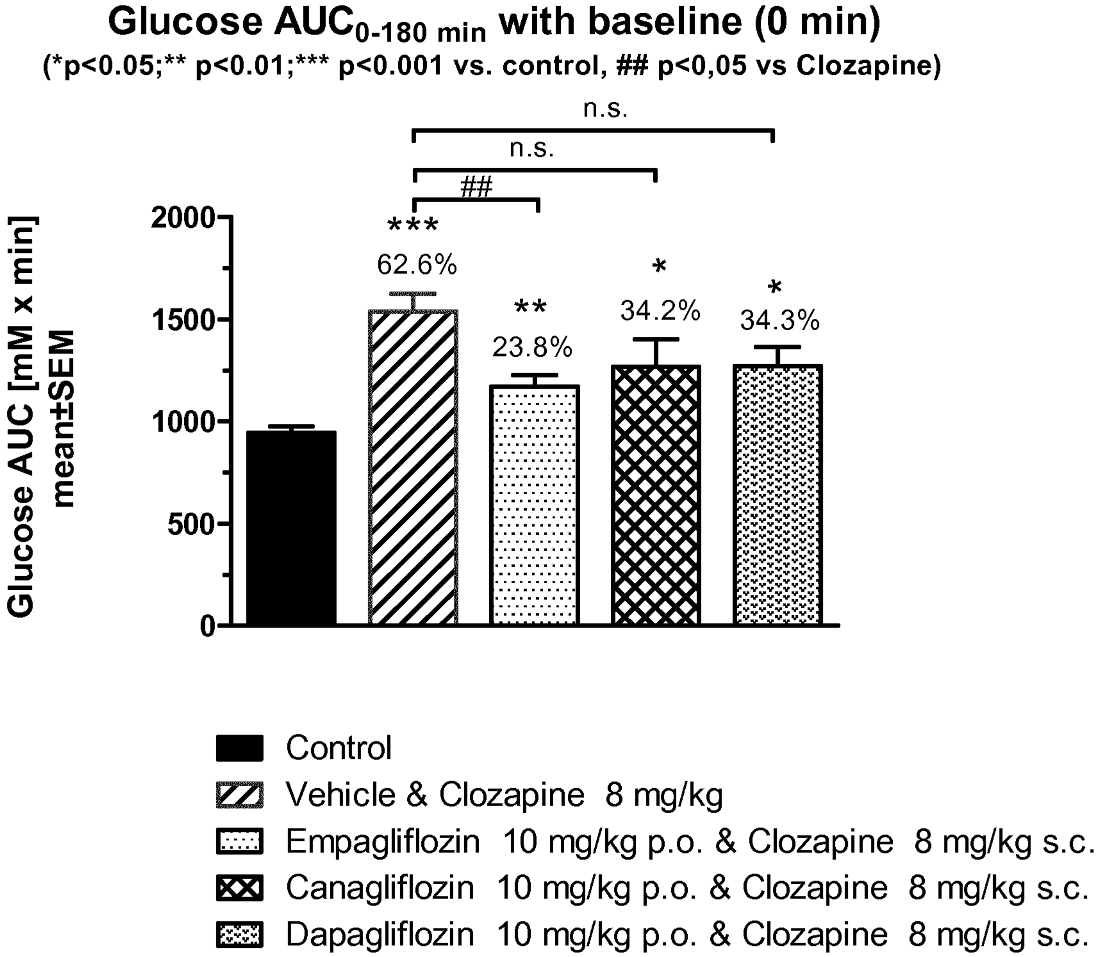
Glucose AUC0-180 min with baseline (0 min)
(*p<0.05;** p<0.01;*** p<0.001 vs. control, ## p<0,05 vs Clozapine)
n.s.
n.s.
##
***
62.6%
**
23.8%
*
34.2%
*
34.3%
2000
1500
1000
500
0
Glucose AUC [mM x min]
mean±SEM
Control
Vehicle & Clozapine 8 mg/kg
Empagliflozin 10 mg/kg p.o. & Clozapine 8 mg/kg s.c.
Canagliflozin 10 mg/kg p.o. & Clozapine 8 mg/kg s.c.
Dapagliflozin 10 mg/kg p.o. & Clozapine 8 mg/kg s.c.

# Figure 4A
## Female SD rats (n=8)
## Oral glucose tolerance test

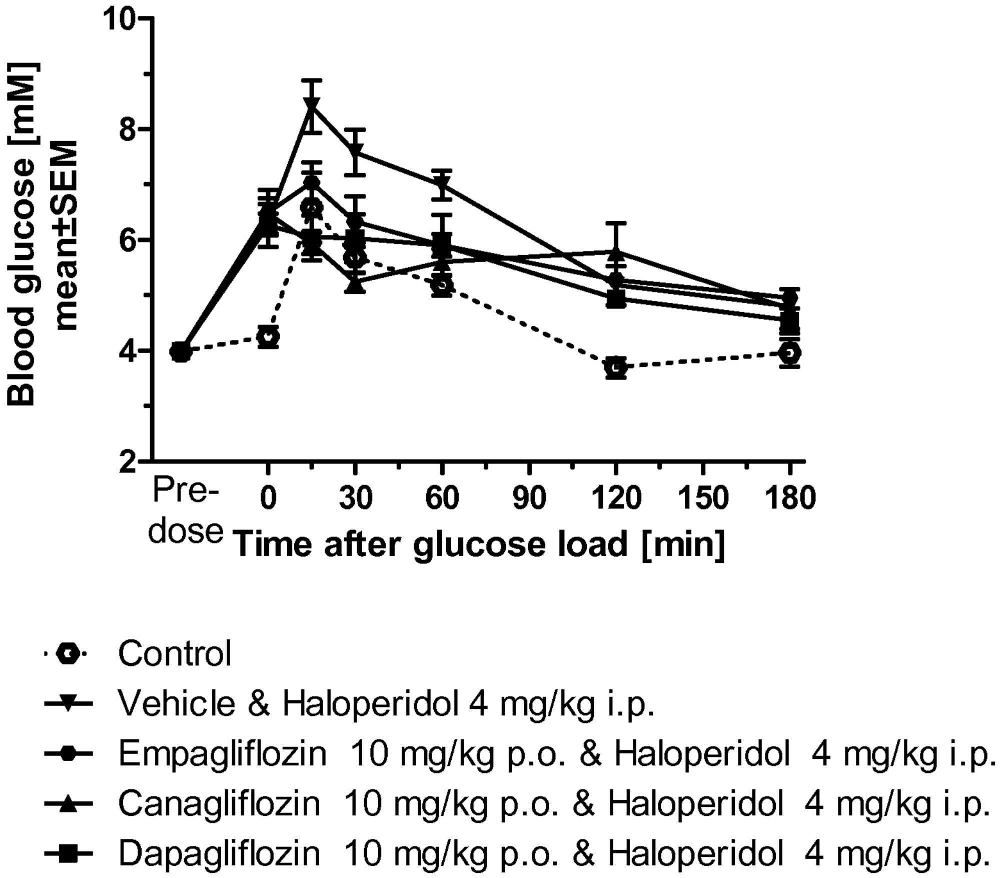

**Figure 4B**

**Glucose $AUC_{0\text{-}180\ min}$ with baseline (0 min)**

**(* p<0.05;** p<0.01; *** p<0.001 vs. Control, # p<0,05vs Haloperidol)**

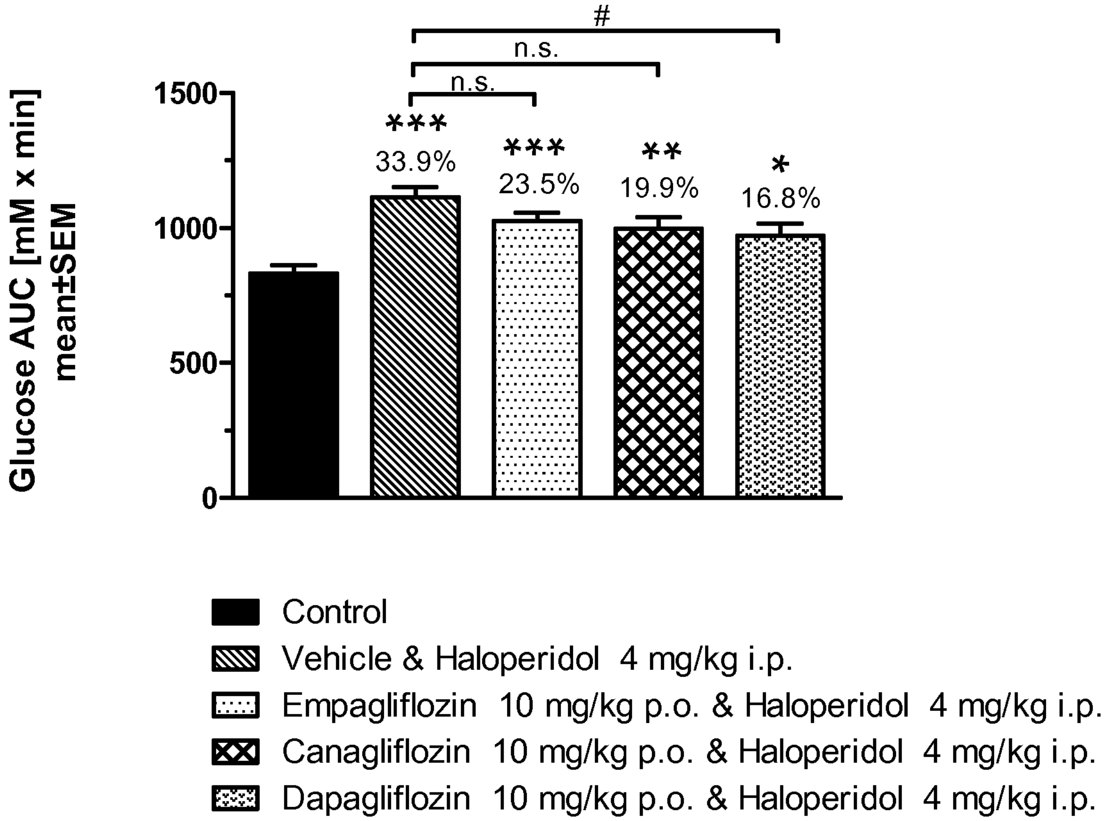

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0127128 A
- WO 03099836 A
- WO 2005092877 A
- WO 2006034489 A
- WO 2006064033 A
- WO 2006117359 A
- WO 2006117360 A
- WO 2007025943 A
- WO 2007028814 A
- WO 2007031548 A
- WO 2007093610 A
- WO 2007128749 A
- WO 2008049923 A
- WO 2008055870 A
- WO 2008055940 A
- WO 2006120208 A
- WO 2008020011 A
- WO 2011039107 A
- WO 2011039108 A
- WO 2008116179 A
- WO 2008002824 A
- WO 2005012326 A
- WO 2009035969 A
- WO 2008069327 A
- WO 2010092126 A


### Non-patent literature cited in the description


- **BOYDA et al.** *Trends in Pharmacological Sciences,* 2000, vol. 31, 484-497
- **FORD ES et al.** *JAMA.,* 2002, vol. 287, 356-9
- **KATSUKI A et al.** *Diabetes Care,* 2001, vol. 24, 362-5
- **MATTHEWS et al.** *Diabetologia,* 1985, vol. 28, 412-19
- **FORST et al.** *Diabetes,* 2003, vol. 52 (1), A459
- **GALVIN P et al.** *Diabet Med,* 1992, vol. 9, 921-8
- **STUMVOLL et al.** *Eur J Clin Invest,* 2001, vol. 31, 380-81
- **J. B. MEIGS et al.** *Diabetes,* 2003, vol. 52, 1475-1484
- *Diabetes Care,* 2002, vol. 25, 742-749
- **LAAKSONEN DE et al.** *Am J Epidemiol,* 2002, vol. 156, 1070-7
- *JAMA: Journal of the American Medical Association,* 2001, vol. 285, 2486-2497
- **LAAKSONEN DE et al.** *Am J Epidemiol.,* 2002, vol. 156, 1070-7
- Labor und Diagnose. TH-Books Verlagsgesellschaft mbH, 2000